(19) Europäisches Patentamt / European Patent Office / Office européen des brevets

(11) EP 2 922 560 B1

(12) **EUROPEAN PATENT SPECIFICATION**

(45) Date of publication and mention of the grant of the patent:
**04.01.2017 Bulletin 2017/01**

(21) Application number: **13792577.2**

(22) Date of filing: **25.10.2013**

(51) Int Cl.:
***C07K 14/715*** *(2006.01)* ***A61K 38/17*** *(2006.01)*
***C07K 16/28*** *(2006.01)*

(86) International application number:
**PCT/CZ2013/000137**

(87) International publication number:
**WO 2014/079399 (30.05.2014 Gazette 2014/22)**

(54) **POLYPEPTIDE ANTAGONISTS OF HUMAN IL-23 RECEPTOR FOR TREATMENT OF AUTOIMMUNE DISEASES**

POLYPEPTIDANTAGONISTEN DES HUMANEN REZEPTORS IL-23 ZUR BEHANDLUNG VON AUTOIMMUNKRANKHEITEN

ANTAGONISTES POLYPEPTIDIQUES DU RÉCEPTEUR DE L'IL-23 HUMAINE POUR LE TRAITEMENT DE MALADIES AUTO-IMMUNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.11.2012 CZ 20120829**

(43) Date of publication of application:
**30.09.2015 Bulletin 2015/40**

(73) Proprietor: **Biotechnologicky Ustav AV CR, v.v.i.**
**Vestec**
**25242 Jesenice u Prahy (CZ)**

(72) Inventors:
- **MALY, Petr**
**14700 Praha 4 (CZ)**
- **SEBO, Peter**
**14200 Praha 4 (CZ)**
- **KUCHAR, Milan**
**69701 Sobulky (CZ)**
- **VANKOVA, Lucie**
**14300 Praha 4 (CZ)**
- **PETROKOVA, Hana**
**19800 Praha 9 (CZ)**
- **OSICKA, Radim**
**14000 Praha 4 (CZ)**

(74) Representative: **Hartvichova, Katerina**
**Inventia s.r.o.**
**Na Belidle 3**
**150 00 Praha 5 (CZ)**

(56) References cited:
**WO-A1-2010/142534 WO-A2-2009/007849**

- **FRANCESCA CAPON ET AL: "Sequence variants in the genes for the interleukin-23 receptor (IL23R) and its ligand (IL12B) confer protection against psoriasis", HUMAN GENETICS, SPRINGER, BERLIN, DE, vol. 122, no. 2, 22 June 2007 (2007-06-22) , pages 201-206, XP019542837, ISSN: 1432-1203, DOI: 10.1007/S00439-007-0397-0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 922 560 B1

SEQUENCE LISTING

<110> Biotechnologický ústav AV ČR, v. v. i.

<120> Polypeptide antagonists of human IL-23 receptor for treatment of autoimmune diseases

<130> P

<160> 51

<170> PatentIn version 3.5

<210> 1
<211> 27
<212> PRT

<213> artificial

<220>
<223> modified ABD sequence

<400> 1

```
Lys Tyr Lys Asn Gly Ile Asn Asn Ala Leu Cys Val Arg Arg Val Lys
1               5                   10                  15

Ala Leu Ile Asp Trp Ile Leu Ala Tyr Leu Pro
            20                  25
```

<210> 2
<211> 27
<212> PRT
<213> artificial

<220>
<223> modified ABD sequence

<400> 2

```
Thr Tyr Lys Asn Asp Ile Asn Ala Ala Ser Tyr Val Pro Ala Val Lys
1               5                   10                  15

Trp Ala Ile Asp Arg Ile Leu Ala Ser Leu Pro
            20                  25
```

<210> 3
<211> 27
<212> PRT
<213> artificial

<220>
<223> modified ABD sequence

<400> 3

```
Tyr Tyr Lys Asn Arg Ile Asn Pro Ala Cys His Val Leu Ser Val Lys
1               5                   10                  15

Ser Asn Ile Asp Trp Ile Leu Ala Ser Leu Pro
            20                  25
```

<210> 4
<211> 27
<212> PRT
<213> artificial

<220>
<223> modified ABD sequence

<400> 4

```
Val Tyr Lys Asn Thr Ile Asn Ile Ala Ile Pro Val Arg Val Val Lys
1               5                   10                  15

Arg Val Ile Asp Trp Ile Leu Ala Val Leu Pro
            20                  25
```

<210> 5
<211> 27
<212> PRT
<213> artificial

<220>
<223> modified ABD sequence

<400> 5

```
Ala Tyr Lys Asn Leu Ile Asn Ala Ala Leu Ile Val Ala Lys Val Lys
1               5                   10                  15

Leu Leu Ile Asp Ala Ile Leu Ala Pro Leu Pro
            20                  25
```

<210> 6
<211> 27
<212> PRT
<213> artificial

<220>
<223> modified ABD sequence

<400> 6

```
His Tyr Lys Asn Trp Ile Asn Pro Ala Arg Arg Val Arg Pro Val Lys
1               5                   10                  15

Trp Leu Ile Asp Ala Ile Leu Ala Ala Leu Pro
            20                  25
```

<210> 7
<211> 27
<212> PRT
<213> artificial

<220>
<223> modified ABD sequence

<400> 7

```
Arg Tyr Lys Asn Ser Ile Asn Arg Ala Leu Pro Val Ala Ala Val Lys
1               5                   10                  15

Trp Ala Leu Asp Leu Ile Leu Ala Trp Leu Pro
```

20 25

<210> 8
<211> 27
<212> PRT
<213> artificial

<220>
<223> modified ABD sequence

<400> 8

Trp Tyr Lys Asn Cys Ile Thr Ala Ala Arg Ala Val Thr Thr Val Lys
1 5 10 15

Leu Leu Ile Asp Thr Ile Leu Ala Leu Leu Pro
20 25

<210> 9
<211> 27
<212> PRT
<213> artificial

<220>
<223> modified ABD sequence

<400> 9

His Tyr Lys Asn Pro Ile Asn Val Ala Trp Thr Val Gly Arg Val Lys
1 5 10 15

Val Trp Ile Asp Ala Ile Leu Ala Pro Leu Pro
20 25

<210> 10
<211> 27
<212> PRT
<213> artificial

<220>
<223> modified ABD sequence

<400> 10

Pro Tyr Lys Asn Pro Ile Asn Cys Ala Cys Pro Val Thr Glu Val Lys
1 5 10 15

Pro Pro Ile Asp Ala Ile Leu Ala Leu Leu Pro
20 25

<210> 11
<211> 27
<212> PRT
<213> artificial

<220>
<223> modified ABD sequence

<400> 11

His Tyr Lys Asn Ser Ile Asn Pro Ala Pro Gln Val Ile Val Val Lys
1 5 10 15

Val Asn Ile Asp Leu Ile Leu Ala Gly Leu Pro
20 25

<210> 12
<211> 27
<212> PRT
<213> artificial

<220>
<223> modified ABD sequence

<400> 12

Arg Tyr Lys Asn Trp Ile Asn Arg Ala Trp Leu Val Ala Leu Val Lys
1 5 10 15

Arg Gln Ile Asp Gln Ile Leu Ala Leu Leu Pro
20 25

<210> 13
<211> 27
<212> PRT
<213> artificial

<220>
<223> modified ABD sequence

<400> 13

Glu Tyr Lys Asn Ala Ile Asn Ala Ala Asn Pro Val Ser Gly Val Lys
1 5 10 15

Arg Pro Ile Asp Val Ile Leu Ala Ala Leu Pro
20 25

<210> 14
<211> 27
<212> PRT
<213> artificial

<220>
<223> modified ABD sequence

<400> 14

His Tyr Lys Asn Ser Ile Asn Pro Ala Phe Lys Val His Ser Val Lys
1 5 10 15

Met Gly Ile Asp Trp Ile Leu Ala Gly Leu Pro
20 25

<210> 15
<211> 27
<212> PRT
<213> artificial

<220>
<223> modified ABD sequence

<400> 15

Trp Tyr Lys Asn Arg Ile Asn Thr Ala Leu Thr Val Ala Cys Val Lys
1 5 10 15

Leu Val Ile Asp Trp Ile Leu Ala Ala Leu Pro
20 25

<210> 16
<211> 46
<212> PRT
<213> Streptococcus sp.

<400> 16

Leu Ala Glu Ala Lys Val Leu Ala Asn Arg Glu Leu Asp Lys Tyr Gly
1 5 10 15

Val Ser Asp Tyr Tyr Lys Asn Leu Ile Asn Asn Ala Lys Thr Val Glu
20 25 30

Gly Val Lys Ala Leu Ile Asp Glu Ile Leu Ala Ala Leu Pro
35 40 45

<210> 17
<211> 19
<212> PRT
<213> artificial

<220>
<223> modified ABD sequence

<400> 17

Leu Ala Glu Ala Lys Val Leu Ala Asn Arg Glu Leu Asp Lys Tyr Gly
1 5 10 15

Val Ser Asp

<210> 18

<211> 19
<212> PRT
<213> artificial

<220>
<223> modified ABD sequence

<400> 18

```
Leu Ala Glu Ala Lys Val Leu Thr Asn Arg Glu Leu Asp Lys Tyr Gly
1               5                   10                  15

Val Ser Asp
```

<210> 19
<211> 19
<212> PRT
<213> artificial

<220>
<223> modified ABD sequence

<400> 19

```
Leu Ala Glu Thr Lys Val Leu Ala Asn Arg Glu Leu Asp Lys Tyr Gly
1               5                   10                  15

Val Ser Asp
```

<210> 20
<211> 46
<212> PRT
<213> artificial

<220>
<223> modified ABD sequence

<400> 20

```
Leu Ala Glu Ala Lys Val Leu Ala Asn Arg Glu Leu Asp Lys Tyr Gly
1               5                   10                  15

Val Ser Asp Lys Tyr Lys Asn Gly Ile Asn Asn Ala Leu Cys Val Arg
            20                  25                  30

Arg Val Lys Ala Leu Ile Asp Trp Ile Leu Ala Tyr Leu Pro
        35                  40                  45
```

<210> 21
<211> 46
<212> PRT
<213> artificial

<220>
<223> modified ABD sequence

<400> 21

Leu Ala Glu Ala Lys Val Leu Ala Asn Arg Glu Leu Asp Lys Tyr Gly
1               5                   10                  15

Val Ser Asp Thr Tyr Lys Asn Asp Ile Asn Ala Ala Ser Tyr Val Pro
            20                  25                  30

Ala Val Lys Trp Ala Ile Asp Arg Ile Leu Ala Ser Leu Pro
        35                  40                  45

<210> 22
<211> 46
<212> PRT
<213> artificial

<220>
<223> modified ABD sequence

<400> 22

Leu Ala Glu Ala Lys Val Leu Ala Asn Arg Glu Leu Asp Lys Tyr Gly
1               5                   10                  15

Val Ser Asp Tyr Tyr Lys Asn Arg Ile Asn Pro Ala Cys His Val Leu
            20                  25                  30

Ser Val Lys Ser Asn Ile Asp Trp Ile Leu Ala Ser Leu Pro
        35                  40                  45

<210> 23
<211> 46
<212> PRT
<213> artificial

<220>
<223> modified ABD sequence

<400> 23

Leu Ala Glu Ala Lys Val Leu Ala Asn Arg Glu Leu Asp Lys Tyr Gly
1               5                   10                  15

Val Ser Asp Val Tyr Lys Asn Thr Ile Asn Ile Ala Ile Pro Val Arg
            20                  25                  30

Val Val Lys Arg Val Ile Asp Trp Ile Leu Ala Val Leu Pro
        35                  40                  45

<210> 24
<211> 46
<212> PRT
<213> artificial

<220>

<223> modified ABD sequence

<400> 24

```
Leu Ala Glu Ala Lys Val Leu Thr Asn Arg Glu Leu Asp Lys Tyr Gly
1               5                   10                  15

Val Ser Asp Ala Tyr Lys Asn Leu Ile Asn Ala Ala Leu Ile Val Ala
            20                  25                  30

Lys Val Lys Leu Leu Ile Asp Ala Ile Leu Ala Pro Leu Pro
        35                  40                  45
```

<210> 25
<211> 46
<212> PRT
<213> artificial

<220>
<223> modified ABD sequence

<400> 25

```
Leu Ala Glu Ala Lys Val Leu Ala Asn Arg Glu Leu Asp Lys Tyr Gly
1               5                   10                  15

Val Ser Asp His Tyr Lys Asn Trp Ile Asn Pro Ala Arg Arg Val Arg
            20                  25                  30

Pro Val Lys Trp Leu Ile Asp Ala Ile Leu Ala Ala Leu Pro
        35                  40                  45
```

<210> 26
<211> 46
<212> PRT
<213> artificial

<220>
<223> modified ABD sequence

<400> 26

```
Leu Ala Glu Ala Lys Val Leu Ala Asn Arg Glu Leu Asp Lys Tyr Gly
1               5                   10                  15

Val Ser Asp Arg Tyr Lys Asn Ser Ile Asn Arg Ala Leu Pro Val Ala
            20                  25                  30

Ala Val Lys Trp Ala Leu Asp Leu Ile Leu Ala Trp Leu Pro
        35                  40                  45
```

<210> 27
<211> 46
<212> PRT
<213> artificial

<220>
<223> modified ABD sequence

<400> 27

```
Leu Ala Glu Ala Lys Val Leu Ala Asn Arg Glu Leu Asp Lys Tyr Gly
1               5                   10                  15

Val Ser Asp Trp Tyr Lys Asn Cys Ile Thr Ala Ala Arg Ala Val Thr
            20                  25                  30

Thr Val Lys Leu Leu Ile Asp Thr Ile Leu Ala Leu Leu Pro
        35                  40                  45
```

<210> 28
<211> 46
<212> PRT
<213> artificial

<220>
<223> modified ABD sequence

<400> 28

```
Leu Ala Glu Ala Lys Val Leu Ala Asn Arg Glu Leu Asp Lys Tyr Gly
1               5                   10                  15

Val Ser Asp His Tyr Lys Asn Pro Ile Asn Val Ala Trp Thr Val Gly
            20                  25                  30

Arg Val Lys Val Trp Ile Asp Ala Ile Leu Ala Pro Leu Pro
        35                  40                  45
```

<210> 29
<211> 46
<212> PRT
<213> artificial

<220>
<223> modified ABD sequence

<400> 29

```
Leu Ala Glu Ala Lys Val Leu Ala Asn Arg Glu Leu Asp Lys Tyr Gly
1               5                   10                  15

Val Ser Asp Pro Tyr Lys Asn Pro Ile Asn Cys Ala Cys Pro Val Thr
            20                  25                  30

Glu Val Lys Pro Pro Ile Asp Ala Ile Leu Ala Leu Leu Pro
        35                  40                  45
```

<210> 30
<211> 46

<212> PRT
<213> artificial

<220>
<223> modified ABD sequence

<400> 30

```
Leu Ala Glu Ala Lys Val Leu Ala Asn Arg Glu Leu Asp Lys Tyr Gly
1               5                   10                  15

Val Ser Asp His Tyr Lys Asn Ser Ile Asn Pro Ala Pro Gln Val Ile
            20                  25                  30

Val Val Lys Val Asn Ile Asp Leu Ile Leu Ala Gly Leu Pro
        35                  40                  45
```

<210> 31
<211> 46
<212> PRT
<213> artificial

<220>
<223> modified ABD sequence

<400> 31

```
Leu Ala Glu Ala Lys Val Leu Ala Asn Arg Glu Leu Asp Lys Tyr Gly
1               5                   10                  15

Val Ser Asp Arg Tyr Lys Asn Trp Ile Asn Arg Ala Trp Leu Val Ala
            20                  25                  30

Leu Val Lys Arg Gln Ile Asp Gln Ile Leu Ala Leu Leu Pro
        35                  40                  45
```

<210> 32
<211> 46
<212> PRT
<213> artificial

<220>
<223> modified ABD sequence

<400> 32

Leu Ala Glu Ala Lys Val Leu Ala Asn Arg Glu Leu Asp Lys Tyr Gly
1 5 10 15

Val Ser Asp Glu Tyr Lys Asn Ala Ile Asn Ala Ala Asn Pro Val Ser
20 25 30

Gly Val Lys Arg Pro Ile Asp Val Ile Leu Ala Ala Leu Pro
35 40 45

<210> 33
<211> 46
<212> PRT
<213> artificial

<220>
<223> modified ABD sequence

<400> 33

Leu Ala Glu Ala Lys Val Leu Ala Asn Arg Glu Leu Asp Lys Tyr Gly
1 5 10 15

Val Ser Asp His Tyr Lys Asn Ser Ile Asn Pro Ala Phe Lys Val His
20 25 30

Ser Val Lys Met Gly Ile Asp Trp Ile Leu Ala Gly Leu Pro
35 40 45

<210> 34
<211> 46
<212> PRT
<213> artificial

<220>
<223> modified ABD sequence

<400> 34

Leu Ala Glu Thr Lys Val Leu Ala Asn Arg Glu Leu Asp Lys Tyr Gly
1 5 10 15

Val Ser Asp Trp Tyr Lys Asn Arg Ile Asn Thr Ala Leu Thr Val Ala
20 25 30

Stránka 10

Cys Val Lys Leu Val Ile Asp Trp Ile Leu Ala Ala Leu Pro
35 40 45

<210> 35
<211> 68
<212> DNA
<213> Artificial

<220>

<223> Forward primer - Example 2

<400> 35

attaccatgg gcagcagcca ccatcatcat catcacagca gcggaattac aaatataaac 60

tgctctgg 68

<210> 36
<211> 34
<212> DNA
<213> Artificial

<220>
<223> Reverse primer, Example 2

<400> 36
gggcacctta cttctgacaa ctgactcgag atat 34

<210> 37
<211> 30
<212> DNA
<213> Artificial

<220>
<223> Forward primer, Example 3

<400> 37
gggctagcta gcagagctgt gcctgggggc 30

<210> 38
<211> 30
<212> DNA
<213> Artificial

<220>
<223> Reverse primer, Example 3

<400> 38
gcgcctcgag gggactcagg gttgctgctc 30

<210> 39
<211> 40
<212> PRT
<213> Artificial

<220>
<223> solubility-supporting sequence, Example 3

<400> 39

Leu Glu Lys Lys Thr Cys Thr Ser Arg Ala Ser Ser Thr Thr Thr Thr
1 5 10 15

Thr Thr Glu Ile Arg Leu Leu Thr Lys Pro Glu Arg Lys Leu Ser Trp
20 25 30

Leu Leu Pro Pro Leu Ser Asn Asn
35 40

<210> 40
<211> 57
<212> DNA
<213> Artificial

<220>
<223> Forward primer, Example 5

<400> 40
ttagctgaag ctaaagtctt agctaacaga gaacttgaca aatatggagt aagtgac 57

<210> 41
<211> 17
<212> DNA
<213> Artificial

<220>
<223> Reverse primer, Example 5

<400> 41
accgcggatc caggtaa 17

<210> 42
<211> 113
<212> DNA
<213> Artificial

<220>
<223> randomized codons, Example 5

<220>
<221> misc_feature
<222> (19)..(20)
<223> n is a, c, g, or t

<220>
<221> misc_feature
<222> (31)..(32)
<223> n is a, c, g, or t

<220>
<221> misc_feature
<222> (40)..(41)
<223> n is a, c, g, or t

<220>
<221> misc_feature
<222> (43)..(44)
<223> n is a, c, g, or t

<220>

<221> misc_feature
<222> (52)..(53)
<223> n is a, c, g, or t

<220>
<221> misc_feature
<222> (55)..(56)
<223> n is a, c, g, or t

<220>
<221> misc_feature
<222> (61)..(62)
<223> n is a, c, g, or t

<220>
<221> misc_feature
<222> (64)..(65)
<223> n is a, c, g, or t

<220>
<221> misc_feature
<222> (70)..(71)
<223> n is a, c, g, or t

<220>
<221> misc_feature
<222> (79)..(80)
<223> n is a, c, g, or t

<220>
<221> misc_feature
<222> (91)..(92)
<223> n is a, c, g, or t

<400> 42

accgcggatc caggtaamnn agctaaaatm nnatctatmn nmnnttttac mnnmnnaacm 60

nnmnnggcmn ngttgatmnn gttcttgtam nngtcactta ctccatattt gtc 113

<210> 43
<211> 57
<212> DNA
<213> Artificial

<220>
<223> Forward primer, Example 5

<400> 43
ttacctggat ccgcggtcgg ttcgagctcc aagcttggat ctggtggcca gaagcaa 57

<210> 44
<211> 36
<212> DNA
<213> Artificial

<220>
<223> Reverse primer, Example 5

<400> 44
tttccgctcg agctacggtt tgaagtccaa tggcgc 36

<210> 45
<211> 64
<212> DNA
<213> Artificial

<220>
<223> Forward primer, Example 5

<400> 45

ttcctccatg ggtatgagag gatcgcatca ccatcaccat cacttagctg aagctaaagt 60

ctta 64

<210> 46
<211> 41
<212> DNA
<213> Artificial

<220>
<223> Primer, Example 5

<400> 46
atacgaaatt aatacgactc actataggga gaccacaacg g 41

<210> 47
<211> 80
<212> DNA
<213> Artificial

<220>
<223> Primer, Example 5

<400> 47

gggagaccac aacggtttcc ctctagaaat aattttgttt aactttaaga aggagatata 60

ccatgggtat gagaggatcg 80

<210> 48
<211> 45
<212> DNA
<213> Artificial

<220>
<223> Primer, Example 5

<400> 48
ccgcacacca gtaaggtgtg cggtttcagt tgccgctttc tttct 45

<210> 49
<211> 81
<212> DNA
<213> Artificial

<220>
<223> Reverse primer, Example 6

<400> 49

tttccgctcg agctattcgt gccattcgat tttctgagcc tcgaagatgt cgttcaggcc 60

cggtttgaag tccaatggcg c 81

<210> 50
<211> 33
<212> DNA
<213> Artificial

<220>
<223> Reverse primer, Example 6

<400> 50
ttactaggat ccaggtaatg cagctaaaat ttc 33

<210> 51
<211> 15
<212> PRT
<213> Artificial

<220>
<223> Biotinylation sequence, Example 6

<400> 51

Gly Leu Asn Asp Ile Phe Glu Ala Gln Lys Ile Glu Trp His Glu
1 5 10 15

## Description

### Field of the Invention

The present invention relates to a novel class of polypeptides, derived from an albumin-binding domain of the streptococcal protein G, that inhibit binding of human IL-23 cytokine to its cognate IL-23 receptor (IL-23R). They are useful as therapeutic or diagnostic agents.

### Background of the Invention

Autoimmune diseases such as psoriasis, Crohn disease, rheumatoid arthritis or multiple sclerosis are known to be associated with the IL-23 cytokine which induces the signaling promoted by IL-23 receptor-expressing $T_H$17 cell or other lymphocyte subsets (Oppmann B et al.: Immunity 2000;13(5):715-725; Cua DJ et al.: Nature 2003;421(6924):744-748; Langrish CL et al.:J Exp Med 2005;201(2):233-240; Chan JR et al.:J Exp Med 2006;203(12):2577-2587; Capon F et al.: Hum Genet 2007;122(2):201-206; Vaknin-Dembinsky A et al.: J Immunol 2006;176(12):7768-7774; Duerr RH et al.: Science 2006;314(5804):1461-1463). In these cell types, the dendritic cell-released IL-23 cytokine, composed of a unique p19 subunit and a common p40 subunit (Beyer BM et al.: J Mol Biol 2008;382(4):942-955; Lupardus PJ et al.: J Mol Biol 2008;382(4):931-941), activates the signaling pathway through an interaction of the p19 subunit with its cognate cell membrane receptor IL-23R and a simultaneous interaction of the p40 subunit with the surface receptor β1 of the cytokine IL-12 (Parham C et al.: J Immunol 2002;168(11):5699-5708; Kastelein RA et al.: Annu Rev Immunol 2007;25:221-242). Synergistic tethering of the IL-23 heterodimer to both receptor units leads to receptor heterodimerization followed by formation of a quarternary complex triggering the signaling cascade of the Jak/Stat pathway, involving the molecules Jak2, Tyk2, Stat1, Stat3, Stat4 and Stat5. Transduction of the signal activates the transcription machinery inducing the secretion of a cocktail of inflammatory modulators such as IL-17A, IL-17F, IL-22 and several chemokines that stimulate keratinocytes and other cell types, thereby playing a pivotal role in the pro-inflammatory process (review Boniface K et al.: Immunol Rev 2008;226:132-146).

Efficient therapeutic intervention, preventing hyper-proliferation of keratinocytes in clinical manifestations of psoriasis, depends on the blockade of interaction between p19/p40 subunits of IL-23 and their cognate cell membrane receptors (Toichi E et al.: J Immunol 2006;177(7):4917-4926; Krueger GG et al.: N Engl J Med 2007;356(6):580-592; Gottlieb AB et al.: Curr Med Res Opin 2007;23(5):1081-1092). Recently it has been demonstrated that the monoclonal antibody-based drug Stelara (ustekinumab, Janssen Biotech), blocking the p40 subunit of the IL-23 cytokine and preventing it from the interaction with the β1 receptor of IL-12 cytokine, reached an excellent efficacy in the treatment of medium and severe forms of psoriasis (Leonardi CL et al.: Lancet 2008;371(9625):1665-1674; Papp KA et al.: Lancet 2008;371(9625):1675-1684). However, this drug inhibits the binding of the common p40 subunit, shared both by IL-12 and IL-23, and thus interferes with two different signaling pathways. This often leads to complications, including cardiovascular side effects or higher risk of cancer development. Novel therapeutic strategies, therefore, require development of novel IL-23R antagonists that will separate IL-23-mediated signaling from the IL-12 cascade, thus preserving $T_H$-1 cell differentiation and immunity. Attention is at present focused on the development of new drugs targeted solely to the IL-23-mediated signaling pathway, such as specific anti-p19 antibodies MK-3222 (Merck), CNTO 1959 (Janssen Biotech) and AMG 139 (Amgen/MedImmune) (Garber K: Nat Biotechnol 2012;30(6):475-477), or targeted antibodies described in the patent applications WO 2008/103432, WO 2010/027766 a WO 2012/093127. However, the antibodies are large molecules, requiring elaborate preparation and showing a time-limited mobility within the organism, in particular with regard to skin and tissue penetrability.

An alternative to conventional monoclonal antibody-based drugs are artificial ligands derived from small protein scaffolds (Binz HK et al.: Current Opinion in Biotechnology 2005;16(4):459-469; Nygren PA et al.: Journal of Immunological Methods 2004;290(1-2):3-28; Gronwall C et al.: J Biotechnol 2009;140(3-4):254-269). Small protein scaffolds arising from modification of amino acid residues of the albumin-binding domain (ABD) of streptococcal protein G were disclosed in US 2008/0187517 in which they were used for increasing the affinity to human serum albumin (HSA). Furthermore, IL-23-binding proteins derived from fibronection are known (WO 2011/103105).

IL-23 receptor belongs to the class-I cytokine receptor family and shares typical features with tandem fibronectin-type III (FnIII) domains containing a hallmark pattern of disulfide bonds and WQPWS sequence tag similar to a conserved WSXWS cytokine receptor consensus located in the transmembrane-proximal FnIII domain (Parham C et al.: J Immunol 2002;168(11):5699-5708). Both domains form a cytokine-binding homology region (CHR) which, in concert with a terminal Ig-like domain, is believed to play a substantial role in IL-23 binding.

The molecular structure of the IL-23/IL-23R complex is not available and, thus, the precise structural mode-of-function of the IL-23/IL-23R complex is unknown. Therefore, designing efficient inhibitors of IL-23 function with a promising therapeutic potential remains cumbersome.

Disclosure of the Invention

The present invention provides polypeptides containing a sequence with at least 80% identity with at least one sequence selected from the group comprising:

KYKNGINNALCVRRVKALIDWILAYLP (SEQ. ID NO. 1)
TYKNDINAASYVPAVKWAIDRILASLP (SEQ. ID NO. 2)
YYKNRINPACHVLSVKSNIDWILASLP (SEQ. ID NO. 3)
VYKNTINIAIPVRVVKRVIDWILAVLP (SEQ. ID NO. 4)
AYKNLINAALIVAKVKLLIDAILAPLP (SEQ. ID NO. 5)
HYKNW1NPARRVRPVKWLIDAILAALP (SEQ. ID NO. 6)
RYKNS1NRALPVAAVKWALDLILAWLP (SEQ. ID NO. 7)
WYKNCITAARAVTTVKLLIDTILALLP (SEQ. ID NO. 8)
HYKNPINVAWTVGRVKVWIDAILAPLP (SEQ ID NO. 9)
PYKNPINCACPVTEVKPPIDAILALLP (SEQ. ID NO. 10)
HYKNS1NPAPQVIVVKVNIDLILAGLP (SEQ. ID NO. 11)
RYKNWINRAWLVALVKRQIDQILALLP (SEQ. ID NO. 12)
EYKNAINAANPVSGVKRPIDVILAALP (SEQ. ID NO. 13)
HYKNSINPAFKVHSVKMGIDWILAGLP (SEQ. ID NO. 14)
WYKNRINTALTVACVKLVIDWILAALP (SEQ. ID NO. 15)

whereas at the C-terminus or at the N-terminus of said sequence is attached a sequence having at least 80% identity with at least one sequence selected from the group comprising:

LAEAKVLANRELDKYGVSD (SEQ ID NO. 17)
LAEAKVLTNRELDKYGVSD (SEQ ID NO. 18)
LAETKVLANRELDKYGVSD (SEQ ID NO. 19).

Said polypeptides are derived from wild-type albumin-binding domain of *Streptococcal* protein G (LAEAKVLANRELDKYGVSDYYKNLINNAKTVEGVKALIDEILAALP; ABD wt; SEQ. ID NO. 16). Within the framework of the invention it was discovered that ABDwt-derived polypeptides are suitable candidates for compounds binding the human cytokine IL-23 receptor and thereby specifically blocking its binding to the IL-23 p19 subunit. ABDwt has a trihelical structure and it was found that in particular sequences derived from helixes 2 and 3 (SEQ ID NO. 1-15) are important for binding to the IL-23 receptor. They should preferably contain at least 20 amino acid residues. The sequence derived from helix 1 (SEQ ID NO. 17-19) or its part can be attached to the N-terminus or C-terminus of the sequence derived from the helices 2 and 3.

The sequences having at least 80% identity to the sequences SEQ ID NO. 1-15, 17-19, can arise from shortening or extending the sequence, and from point mutations of the amino acid residues.

The association and dissociation equilibrium of the binding the polypeptide (P) of the present invention to the IL-23 receptor (L) to form the complex (C) can be expressed as C $\rightleftarrows$ P + L, and it is characterized by affinity binding constant $K_d$ calculated in accordance with the formula $K_d = \frac{[P]\,[L]}{[C]}$ (wherein [P] is the concentration of P, [L] is the concentration of L a [C] is the concentration of C expressed in mol/l), the affinity binding constant preferably having a value of less than $10^{-7}$ M. This affinity binding constant can be determined by known methods which yield equivalent results. A suitable method can be, e.g., surface plasmon resonance (SPR).

In a preferred embodiment, the polypeptide is selected from the group comprising sequences SEQ ID NO. 1 to SEQ ID NO. 15, having a sequence selected from the group comprising SEQ ID NO. 17 to SEQ ID NO. 19 attached onto the C-terminus or onto the N-terminus.

The present invention further provides a DNA sequence selected from the group comprising complementary DNA coding for the amino acid sequence of the polypeptides of the present invention, and DNA hybridizing with said complementary DNA under conditions of high stringency. Conditions of high stringency refer to the following conditions for washing-off a labelled probe: washing solution containing 0.5 x SSC + 0.1% SDS at the temperature of 60 °C.

The present invention further includes the use of said DNA sequence for the preparation of polypeptides or recombinant proteins produced in bacterial, yeast, insect, mammal or human host cells, and also these host cells, containing at least one DNA sequence of the present invention.

The polypeptides of the present invention are suitable for use in medicine, namely in the treatment of autoimmune diseases. Particularly therapeutically suitable are the polypeptides containing at least one of the sequences SEQ ID NO. 1 to 7, most preferred are the polypeptides containing at least one of the sequences SEQ ID NO. 3, 4 and 6.

Furthermore, the polypeptides of the present invention are suitable for use as diagnostic agents, in particular for autoimmune diseases. For this purpose, it is useful to attach a chromofor or a fluorofor to the polypeptides of the present invention.

Autoimmune diseases are diseases including pro-inflammatory processes promoted by IL-23, and include in particular psoriasis, Crohn disease, rheumatoid arthritis and multiple sclerosis.

The polypeptides of the invention inhibit the binding of the p19 subunit to the IL-23 receptor by interacting with the receptor, thereby specifically blocking the pro-inflammatory signaling pathway. Thus, they can be used as next-generation drugs for the treatment of autoimmune diseases. This inhibitory ability was show in *in vitro* binding assays, competition experiments and in *ex vivo* functional assay. Furthermore, the sequences of the present invention show a decreased affinity towards serum albumins. Novel ABD-derived binders, called REX ligands, are capable of blocking the human cytokine IL-23 receptor function. As these polypeptides exhibit a significant inhibitory effect on IL-23-driven *ex vivo* expansion of primary human Th-17$^+$ cells, they represent unique IL-23R antagonists that can be used for development of innovative therapeutics important for the treatment of autoimmune diseases and also function as improved diagnostic agents. The invention further provides a DNA sequence encoding for such polypeptides and host cells containing such sequence.

Brief Description of the Figures

Figure 1. Recombinant extracellular IL-23 receptor binds the recombinant **p19** subunit of IL-23. The extracellular portion (residues 24 to 350) of human IL-23 receptor was expressed in *E. coli* SHuffle cells, purified and refolded from 8 M urea extracts of inclusion bodies and immobilized on ELISA plates. Binding of soluble DH-MBP-p19 fusion protein or of refolded DH-p19 protein to immobilized receptor was detected by anti-human IL-23 (p19) polyclonal antibody sandwich with secondary anti-IgG-HRP. Error bars represent standard deviations.

**Figure 2. Location of randomized positions in the ABD scaffold.** The protein structure of the ABD domain of streptrococcal protein G (PDBID 1GJT) is shown in ribbon representation, with the $C_\alpha$ positions of the 11 residues selected for randomization shown as yellow spheres.

**Figure 3. Similarity tree of polypeptide sequences of the obtained REX variants binding exIL-23R.** Sequence analysis of 34 cloned REX binders of exIL-23R obtained by ribosome display selection revealed 18 unique sequence variants. For similarity analysis, only the sequences between residues 20 and 46 were compared, as the N-terminal amino acid positions 1-19 were non-randomized.

**Figure 4.** (a) Binding of REX variants to immobilized exIL-23R receptor using ELISA. Receptor was produced in *E. coli* SHufle strain and refolded after extraction in 8M urea solution. Interaction was detected by streptavidin conjugated to horseradish peroxidase. (b) Binding of exIL-23R receptor to immobilized REX variants. Interaction was detected by polyclonal antibody against human receptor exIL-23R (anti-human IL-23R) and a secondary antibody conjugate with horseradish peroxidase (anti-goat IgG-HRP). Graphs show direct binding of the REX ligands to the human IL-23 receptor in comparison to the original unmutated ABDwt molecule.

**Figure 5. REX binders compete with the soluble recombinant p19 subunit of IL-23 for binding to immobilized exIL-23R.** REX-TolA-AVI variants were serially diluted in PBST solution containing 20 nM DH-MBP-p19 as ligand of immobilized exIL-23R. Bound p19 was detected with anti-IL-23 (p19) polyclonal antibody sandwich with secondary anti-IgG-HRP. ABDwt-TolA-AVI, recognizing human serum albumin, served as a negative control. Error bars are shown as standard deviations.

Figure 6. **REX** binders compete with **p19** subunit-mediated binding of IL-23 to the IL-23R-IgG receptor chimera. IL-23R-IgG receptor chimera, produced as folded, soluble and glycosylated protein in eukaryotic cells, was immobilized on ELISA plate and serially diluted inhibitory REX-TolA-AVI ligands were used to compete for binding with 20 nM of DH-MBP-p19 (a) or human 23 mM IL-23 cytokine (b). Bound p19 was detected with anti-IL-23 (p19) polyclonal antibody sandwich with secondary anti-IgG-HRP. ABDwt-TolA-AVI, recognizing human serum albumin, served as a negative control. Error bars represent standard deviations.

Figure 7. Thermal stability of **REX-TolA-AVI** binders measured by fluorescence-based thermal-shift assay. Normalized fluorescences as melting temperature profiles (a) and derivations of normalized fluorescences (b) revealed the Tm points at 50, 56 and 56.5°C for the REX009, REX125 and REX 128, respectively.

**Figure 8. Immobilized REX ligands bind the soluble IL-23R-IgG receptor chimera.** The REX-TolA-AVI variants REX009 and REX125, or ABDwt-TolA-AVI ($ABD_{WT}$), were attached to the surface of SPR sensor over which the IL-23R-IgG receptor chimera was passed at 50 nM concentration. SA indicates sodium acetate buffer.

**Figure 9. Binding of REX variants to human cell lines correlates with IL-23R expression levels. (a)** Expression of IL-23 receptor molecules on the surface of cultured human K-562 and THP-1 cells was detected by flow cytometry using anti-human IL-23R-APC or isotype control-APC mAbs. **(b)** Binding of REX-TolA-AVI variants to K-562 and THP-1 cells. The cells were incubated *with in vivo* biotinylated REX-TolA-AVI proteins or negative ABD-TolA-AVI controls (ABDwt, ΔABD) and the cell-bound proteins were stained with streptavidin-phycoerythrin and analyzed by flow cytometry. In the n.p. control, the REX binders and ABD controls were omitted. (c) Comparison of IL-23 receptor expression on K-562 and Jurkat cell surface. The cells were stained with anti-human IL-23R-APC or isotype-APC control mAbs and analyzed by flow cytometry. (d) Comparison of REX ligand binding to K-562 and Jurkat cells. The cells were incubated with biotinylated REX variants or ABDwt control and cell-bound proteins were detected with streptavidin-phycoerythrin by flow cytometry.

**Figure 10. REX inhibitory variants compete with the p19 subunit of IL-23 for binding to THP-1 cells. (a)** Comparison of DH-p19 protein binding to THP-1 and K-562 cells. Cell-bound DH-p19 molecules were detected by flow cytometry using a mouse anti-IL-23 mAb goat anti-mouse IgG antibody sandwich labeled with Cy5. **(b)** Micromolar concentrations of DH-p19 block binding of REX variants to THP-1 cells. The cells ($2x10^5$) were preincubated with indicated concentrations of DH-p19 before biotinylated REX-TolA-AVI or ABDwt-TolA-AVI control were added to a final concentration of 13 nM and incubation was continued for 30 minutes at 4°C. After washing of cells, the bound ligands were detected by flow cytometry using streptavidin-phycoerythrin. The results are representative of two independent but confirming experiments. Measured values for each of REX variants were supplemented by the particular polynomial trendline.

**Figure 11. REX binders inhibit IL-23-dependent *ex vivo* expansion of IL-17-producing CD4+ T-cells.** PBMCs from 7 healthy volunteers were activated using anti-CD3 and co-stimulation in the presence of IL-23 and IL-2 and in the presence or absence of indicated REX ligands for 3 days. **(a)** Detection of IL-17-producing cells that were gated using FSC, SSC, $CD3^+$ and $CD4^+$. The number in the right-hand bottom corner indicates the percentage of the gated cell population producing solely IL-17 (IFN-γ secretion marks $T_H$-1 type response). **(b, c)** The amount of IL-17-producing cells recovered from PBMC **(b)** or separated T-cell **(c)** samples of healthy donors after 3 days of activation in the presence or absence of the REX ligands. Total cell numbers were normalized to cell counts in IL-23/IL-2-treated samples. Boxes range from 25th to 75th percentiles with a line at the median. Whiskers reach 10th and 90th percentiles. Outlying values are indicated as individual circles.

Examples of carrying out the Invention

Example 1

**Antibodies and detection agents**

Monoclonal antibodies (mAbs) anti-human IL-23R-allophycocyanin (APC) (mouse IgG2B) specific for the human IL-23 receptor and IgG2B isotype control-APC (mouse IgG2b) were obtained from R&D Systems, Minneapolis, MN. Mouse anti-p19 mAb was purchased from Biolegend, San Diego, CA. Cy5-conjugated goat anti-mouse IgG ($F(ab)_2$ fragment) was obtained from Jackson ImmunoResearch Laboratories, West Grove, PA. Streptavidin-phycoerythrin was purchased from eBioscience, San Diego, CA.

**Cell lines and growth conditions**

The cell lines used in the experiments were a human acute monocytic leukemia cell line, THP-1 (ATCC number: TIB-202), a human leukemic cell line, K-562 (ATCC number: CCL-243) and a human T-cell lymphoma cell line, Jurkat (ATCC number: TIB-152). The cells were grown in RPMI-1640 medium (Sigma-Aldrich, St. Louis, MO) supplemented with 10% fetal calf serum (FCS) (GIBCO, Grand Island, N.Y.) and antibiotic antimycotic solution (ATB) (Sigma-Aldrich, St. Louis, MO).

Example 2

**Production of recombinant human IL-23R protein**

The human IL-23 receptor gene consists of 10 exons that code for a 629 amino acid-long transmembrane receptor molecule. For the purpose of being used as a target in ribosome display selection of ABD scaffold-derived ligands, we produced a recombinant form of the extracellular domain of IL-23R (exIL-23R) comprising the residues 24 to 350. This part of IL-23R contains five cysteine pairs encoded within sequences forming both fibronectin type III and terminal Ig-like domains. Therefore, the *E. coli* SHuffle strain supporting formation of disulphide bridges in bacterial cytoplasm was used to express the N-terminally $His_6$-tagged IL-23R (H-exIL-23R). cDNA coding for the extracellular part (fragment Gly24-Asn350) of the human IL-23 receptor (IL-23R, GenBank: AF461422.1) was amplified by PCR using forward primer IL23Rex-F-Nco-his (ATTACCATGGGCAGCAGCCACCATCATCATCATCACAGCAGCGGAATTACA AATATAAACTGCTCTGG), containing the start codon and the $His_6$-tag sequence, and a reverse primer IL23Rex-R-Xho(GGGCACCTTACTTCTGACAACTGAC TCGAGATAT) bearing the TGA stop codon. The resulting PCR product was inserted into the pET-28b vector (Novagen, Germany) using *Nco*I and *Xho*I cloning sites and introduced in *E. coli* TOP10 cells. The obtained plasmid was used for exIL-23R protein production in *E. coli* SHuffle strain (SHuffle® T7 Express Competent *E. coli,* New England Biolabs, Ipswich, MA). Bacteria were grown in liquid LB media with kanamycin (60 $\mu$g/l) at 30°C, induced with 1 mM isopropyl-$\beta$-D-thiogalactopyranoside (IPTG). The exIL-23R protein was extracted from isolated inclusion bodies with 8 M urea in TN buffer (50 mM Tris, 150 mM NaCl, pH = 8.0) and purified by Ni-NTA affinity chromatography.

Alternatively, we cloned the same receptor cDNA fragment into the pET-26b vector (Novagen, Germany) containing a periplasm-targeting pelB leader sequence (pelB-exIL-23R) and expressed the receptor in *E. coli* BL21 ($\lambda$DE3) strain. The LB broth culture with kanamycin (60 $\mu$g/l) was grown at 30°C to reach cell density $OD_{600}$ = 1.0, protein production was induced by 1 mM IPTG, and protein was harvested after 4 hours. However, the protein was also produced as insoluble fraction and was, therefore, refolded from urea-containing extracts.

Both proteins were affinity-purified by Ni-NTA chromatography and refolded by dilution from urea-solutions prior to use in ELISA binding assays and ribosome display selection. The identity of the proteins was confirmed by Western blots using anti-His and anti-IL-23R antibodies.

Example 3

**Production of recombinant human p19 proteins**

For studies of the interactions between IL-23R and IL-23 cytokine, we also produced a recombinant form of the 170 amino acid residue-long p19 subunit of human IL-23. A 23 kDa fusion p19 protein, consisting of a mature portion of p19 with an N-terminal double-polyhistidinyl tag (DH-p19), was produced in fusion to an N-terminal double-$His_6$-TEV purification tag containing the TEV protease consensus cleavage site. Synthetic, codon-optimized p19 cDNA (GENEART, Germany) was inserted into the pET-28b vector as a *Nco*I-*Xho*I fragment and the DH-p19 protein was produced in *E.coli* BL21 ($\lambda$DE3) host cells. Cells were harvested by centrifugation (6,000xg, 20 min), washed with TN buffer (50 mM Tris buffer with 150 mM NaCl, pH = 8) and centrifuged again (5,000xg, 10 min). Pelets were resuspended in 10 ml TN buffer and disrupted by MISONIX 3000 sonicator. The lysates were centrifuged at 40,000xg for 20 min and the insoluble DH-p19 was extracted from inclusion bodies with 2 ml of 8M Urea in TN buffer. The urea extract was left shaking for 1 h at room temperature and centrifuged at 40,000xg for 20 min. The supernatant was applied on 1 ml Ni-NTA agarose column equilibrated with 5 ml of TN buffer. The washing was performed with 10 ml of TN buffer with 8M urea and, subsequently, with TN buffer containing 8M urea and 20 mM imidazole. The DH-p19 was eluted with TN buffer containing 8M urea and 250 mM imidazole in 0.5 ml fractions.

Alternatively, soluble p19 was produced in the form of a fusion protein with maltose-binding protein (MBP) cDNA (*Mal E*) carrying double $His_6$-tag at the N-terminus (DH-MBP-p19, calculated Mw 69 kDa). The p19 sequence was inserted into the assembled pET28b-derived vector, carrying the sequence coding for double $His_6$-MBP-TEV-MCS-TEV-6xHis, using primers p19-F-NheI (GGGCTAGCTAGCAGAGCTGTGCCTGGGGGC) and p19-R-XhoI (GCGCCTCGAGGGGACTCAGGGTTGCTGCTC). *E. coli* TOP 10 (Life Technologies, Carlsbad, CA) host cells were transformed by the p19-cloned vector and plated on LB-agar supplemented with 60 $\mu$g/ml kanamycin. The protein was produced in *E.coli* BL21(DE3). A single colony was used to inoculate 50 ml LB medium containing 60 $\mu$g/ml kanamycin). 20 ml o/n culture was used to inoculate 1 1 of culture medium and grown at 37 °C to OD = 0.6. Culture was induced with IPTG at 20 °C and let to grow for 4h. Cells were harvested by centrifugation at 6,000xg for 20 min, washed with TN buffer (pH = 8) and centrifuged at 5,000xg for 10 min. Pelets were resuspended in 10 ml of TN buffer and disrupted by ultrasound pulses on MISONIX 3000 sonicator. The lysates were centrifuged for 20 min at 40,000xg. The cytoplasmatic fraction containing soluble DH-MBP-p19 was loaded on column with Ni-NTA agarose on ÄKTA purifier (GE Healthcare, UK) on 5ml His-Trap column. Elution was done in three steps with buffer containing 250, 500 and 1000 mM imidazole. 1.5 ml fractions from the elution with 500mM imidazole were inspected on SDS-PAGE and those with DH-MBP-p19 were collected and used further. Its identity was confirmed by detection with anti-IL-23 antibody on Western blots.

In search for solubility-mediating modifications of the p19 protein using several different solubility-supporting fusion tags, the maltose-binding protein (MBP) in combination with a C-terminal modification were found to support the solubility of the produced p19-MBP fusion protein as the only found variant. In this case, the 69 kDa N-terminally double-$His_6$-MBP-p19 fusion protein contained a C-terminal prolongation, installing an additional 40 aa solubility-supporting sequence (LEKKTCTSRASSTTTTTTEIRLLTKPERKLSWLLPPLSNN). Yet this modification was found unintentionally; all other tested C-terminal modifications, including removal of this sequence by a stop codon termination and replacement by a single-his tag, Streptag or FLAG tag sequence consensus, converted the DH-MBP-p19 protein into the insoluble form. Therefore, this soluble version of p19 was used for further studies as an affinity purified product.

Example 4

## Test of the binding activity of IL-23R and p19 by ELISA

To verify that the recombinant refolded exIL-23R protein bound the recombinant form of the p19 subunit, ELISA experiments with immobilized exIL-23R or p19 were performed. The recombinant extracellular portion of the IL-23 receptor (exIL-23R) or the p19 protein were immobilized directly on the NUNC Polysorp 96-well plate surface in coating buffer (100 mM bicarbonate/carbonate solution, pH=9.6) at a concentration of 5-10μg/ml at ~7°C overnight. The plate was washed with PBS buffer containing 0.05% Tween (PBST) and blocked by 1% BSA in the same buffer (PBSTB). Serial dilutions of purified DH-MBP-p19 or DH-p19 recombinant proteins were prepared in PBSTB buffer and p19 binding was detected using mouse anti-human IL-23 (anti-p19) polyclonal antibody followed by goat anti-mouse IgG horseradish peroxidase (HRP) conjugate (BioLegend, San Diego, CA), both diluted in PBSTB 1:1000. exIL-23R binding to immobilized DH-MBP-p19 was detected using goat anti-IL-23R polyclonal antibody (1:250) followed by secondary rabbit anti-goat HRP conjugate (1:1000) (R&D Systems, Minneapolis, MN). OPD substrate (Sigma-Aldrich, St. Luis, MO) was used as HRP substrate in citrate buffer (3.31% sodium citrate tribasic dihydrate, phosphoric acid, pH = 5.0), reactions were stopped with 2 M sulfuric acid and absorbance was read at 492 nm.

As shown in Figure 1, both the refolded DH-p19 protein and the soluble purified DH-MBP-p19 specifically bound to immobilized H-exIL-23R in a saturable manner. This was further confirmed in a reversed setup where the coated DH-MBP-p19 or refolded DH-p19 protein bound the refolded soluble H-exIL-23R protein. These data suggested that the refolded recombinant exIL-23R protein maintained the capacity to specifically bind the p19 subunit of IL-23. Therefore, this protein could be used as a target in ribosome display for selection of IL-23R-specific binders derived from the ABD scaffold.

Example 5

## ABD library construction

Recently we have demonstrated that randomization of 11 residues in the ABD domain scaffold was sufficient for the identification of binders of human interferon-γ with nanomolar range of affinity (Ahmad JN et al.: Proteins 2012;80(3):774-789). We, therefore, used the same approach for the generation of a combinatorial ABD library of a theoretical complexity of $10^{14}$ protein variants (Fig. 2). HPLC-purified synthetic oligonucleotides were used. The forward primer ABDLIB-setBlc (5'-TTAGCTGAAGCTAAAGTCTTAGCTAACAGAGAACTTGACAAATATGGAGTA AGTGAC-3') and the reverse primer setB-rev (5'-ACCGCGGATCCAGGTAA-3') were used for PCR. The latter had distinct codons randomized at defined positions (5'-ACCGCGGATCCAGGTAAMNNAGCTAAAATMNNATCTATMNNMNNTTTTAC MNNMNNAACMNNMNNGGCMNNGTTGATMNNGTTCTTGTAMNNGTCACTT ACTCCATATTTGTC-3'), in which M represents C/A, and N any nucleotides out of A, G, C or T. To serve as a protein spacer for ribosome display, the *tolA* gene (GENE ID: 946625 tolA) was amplified from *E. coli* K12 strain genomic DNA, using the primer pairs ABDLIB-tolA-link (5'-TTACCTGGATCCGCGGTCGGTTCGAGCTCCAAGCTTGGATCTGGTGGCCAGA AGCAA-3') and tolArev (5'-TTTCCGCTCGAGCTACGGTTTGAAGTCCAATGGCGC-3'). The obtained products were linked to the randomized ABD sequences using amplification with primer pairs EWT5-ABDfor1 (5'-TTCCTCCATGGGTATGAGAGGATCGCATCACCATCACCATCACTTAGCTGAA GCTAAAGTCTTA-3') and tolArev. To add the T7 promoter and ribosome binding site sequences, the obtained DNA fragment was subjected to further PCR amplifications with the set of primers T7B (5'-ATACGAAATTAATACGACTCACTATAGGGAGACCACAACGG-3'), SD-EW (5'-GGGAGACCACAACGGTTTCCCTCTAGAAATAATTTTGTTTAACTTTAAGAAG GAGATATACCATGGGTATGAGAGGATCG-3') and *tolA*k *(5'*-CCGCACACCAGTAAGGTGTG CGGTTTCAGTTGCCGCTTTCTTTCT-3'), generating a DNA library of ABD variants lacking the downstream stop codon. The assembled library was *in vitro* transcribed/translated in a single step reaction using *E. coli* extract (EasyXpress Protein Synthesis Mini Kit, QIAGEN, Germany) and used for the selection of translated binders in ribosome display screening.

Example 6

**Ribosome display selection of human IL-23R binders (REX binders)**

For the selection of REX binders, wells of Maxisorp plates (NUNC, Denmark) were coated with decreasing concentrations of recombinant H-exIL-23R protein (round 1 and 2: 25 µg/ml, round 3: 10 µg/ml, round 4: 4 µg/ml, round 5: 1 µg/ml) and blocked with 3% BSA. Pre-selection was performed in wells coated with BSA only. The plate wells were washed three times with TBS (50 mM Tris-HCl pH 7.4, 150 mM NaCl), followed by 10 times washing with cold WBT (50 mM Tris-acetate, pH 7.0, 150 mM NaCl, 50 mM MgAc with increasing concentrations of Tween-20 (round 1 and 2: 0.05%, round 3: 0.25%, round 4: 0.5%, round 5: 1%). To release mRNA from the bound ribosome complex, elution with elution buffer (50 mM Tris-acetate, pH7.5, 150 mM NaCl, 50 mM EDTA) containing 50 µg/ml of *S. cerevisiae* RNA as a carrier was performed. Purified RNA was transcribed into cDNA using specific reverse transcription with the setB-rev reverse primer, and annealing to the 3'end of the ABD cDNA. Double-strand DNA was next obtained by PCR using EWT5-ABDfor1 and setB-rev primers. The final amplified DNA encoding selected ABD variants contained T7 promoter and RBS sequences and a truncated *tolA* fragment.

Two groups of ABD binder cDNA libraries, obtained by reverse transcription after the third or the fifth round of a ribosome display selection campaign, were obtained and cloned as *Nco*I and *Xho*I fragments in the pET-28b vector containing an in-frame inserted full length *tolA* DNA sequence.

To produce *in vivo* biotinylated ABD variants required for verification of their binding affinity to H-exIL-23R by ELISA, REX variants found after 3- or 5-round selection campaigns were modified by installing the AviTag *in vivo* biotinylation sequence (GLNDIFEAQKIEWHE), added downstream of TolA C-terminus. The AviTag sequence for protein detection by streptavidin was introduced using PCR with forward primer EWT5-ABDfor1 and reverse primer tolA-AVIrev1 (TTTCCGCTCGAGCTATTCGTGCCATTCGATTTTCTGAGCCTCGAAGATGTCG TTCAGGCCCGGTTTGAAGTC-CAATGGCGC). The final $His_6$-REX-TolA-AVI fusion proteins were produced as biotinylated proteins in the *Escherichia coli* BL21 (λDE3) BirA strain expressing biotin ligase (BirA) in the presence of 50 µM d-biotin in the LB medium and following induction with 2 mM IPTG. The soluble proteins were purified from cell extracts on Ni-NTA agarose columns.

For all binding assays, a fusion protein containing the original albumin-binding domain (ABDwt) fused with a TolA moiety was used as a negative control. To construct ABDwt with TolA and AviTag sequences (6xHis-ABDwt-TolA-AVI), PCR amplification with ABDwt-tolA plasmid DNA as a template and forward primer EWT5-ABDfor1 and reverse primer ABDrev (TTACTAGGATCCAGGTAATGCAGCTAAAATTTC) was used. The amplified PCR product was digested by NcoI and BamHI enzymes and ligated into pET-28b vector carrying tolA-AVI sequence downstream of BamHI and the resulting fusion His6-ABDwt-TolA-AVI control protein was produced.

Example 7

**Screening of IL-23R-binding REX variants by ELISA**

For binding assays, selected clones were picked, the inserted sequence was verified by restriction analysis (NcoI, BamHI, XhoI) and by DNA sequencing. The clones containing the right sequence 6xHis-ABD-TolA-AviTag were used for isolation of the plasmid DNA and subsequent transformation into the *E. coli* BL21 (λDE3) BirA strain. For the ELISA analysis of the produced modified ABD variants, clone cell lysate prepared as previously described (Ahmad JN et al.: Proteins 2012;80(3):774-789) or the REX proteins purified on Ni-NTA columns were used. Two different sandwich layouts were used for the binding assays. In the first case, NUNC Polysorp plates were coated directly with exIL-23R protein (5 µg/ml, recombinant variant produced in *E. coli* SHuffle strain) in coating buffer at low temperature (~7°C) overnight and the washed plates were blocked by PBSTB. The serially diluted cell lysates or purified REX proteins were applied in PBSTB and the amount of bound biotinylated REX proteins was detected using streptavidin Poly-HRP conjugate (1:1000). In the second setup, the plates were coated with streptavidin (1 µg/ml) in coating buffer, and biotinylated REX proteins (5µg/ml) were immobilized through binding to streptavidin. Serially diluted exIL-23R in PBSTB was added into the wells and receptor binding to immobilized REX variants was detected by goat anti-IL-23Rex polyclonal antibody (1:250) sandwich with secondary rabbit anti-goat HRP conjugate (1:1000, R&D Systems, Minneapolis, MN).

Example 8

**Sequence analysis and clustering of selected REX variants**

Using ribosome display screening, we identified a collection of binders raised against recombinant H-exIL23R. ELISA-positive cell lysates of REX-TolA-AVI clones were selected for further analysis using Western blot. DNA sequences of 34 REX binders were sequentially analyzed and amino acid multiple sequence alignment of all selected clones and construction of the similarity tree were performed using the ClustalW program. The tree is presented as a phenogram rendered by the Phylodendron online service (http://iubio.bio.indiana). As shown in Figure 3, we identified 18 different REX variants, but displaying significant sequence redundancy. All 18 biotinylated REX-TolA-AVI proteins were purified on Ni-NTA agarose and the binding affinity to both immobilized H-exIL-23R and pelB-H-exIL-23R receptor proteins was analyzed using ELISA (Fig.4 a,b). Analysis of several ELISA-negative REX-TolA-AVI variants by restriction digestion and DNA sequencing revealed that these clones lack the ABD cDNA sequence, thus forming the spliced REX version $His_6$-TolA-AVI. One of such clones, called ΔABD, was purified and used for further experiments as another important negative control over the original $His_6$-ABDwt-TolA-AVI.

Example 9

**Identification of inhibitory REX variants**

Based on the results of binding assays performed for all 18 different REX-TolA-AVI variants, 15 clones of the complete REX collection (sequences given below with the randomized binding sequence printed in bold) were selected for further characterization:

LAEAKVLANRELDKYGVSD**KYKNGINNALCVRRVKALIDWILAYLP** (SEQ. ID NO. 20) (REX001)
LAEAKVLANRELDKYGVSD**TYKNDINAASYVPAVKWAIDRILASLP** (SEQ. ID NO. 21) (REX005)
LAEAKVLANRELDKYGVSD**YYKNRINPACHVLSVKSNIDWILASLP** (SEQ. ID NO. 22) (REX009)
LAEAKVLANRELDKYGVSD**VYKNTINIAIPVRVVKRVIDWILAVLP** (SEQ. ID NO. 23) (REX115)
LAEAKVLTNRELDKYGVSD**AYKNLINAALIVAKVKLLIDAILAPLP** (SEQ. ID NO. 24) (REX122)
LAEAKVLANRELDKYGVSD**HYKNWINPARRVRPVKWLIDAILAALP** (SEQ. ID NO. 25) (REX125)
LAEAKVLANRELDKYGVSD**RYKNSINRALPVAAVKWALDLILAWLP** (SEQ. ID NO. 26) (REX128)
LAEAKVLANRELDKYGVSD**WYKNCITAARAVTTVKLLIDTILALLP** (SEQ. ID NO. 27) (REX129)
LAEAKVLANRELDKYGVSD**HYKNPINVAWTVGRVKVWIDAILAPLP** (SEQ ID NO. 28) (REX012)
LAEAKVLANRELDKYGVSD**PYKNPINCACPVTEVKPPIDAILALLP** (SEQ. ID NO. 29) (REX016)
LAEAKVLANRELDKYGVSD**HYKNSINPAPQVIVVKVNIDLILAGLP** (SEQ. ID NO. 30) (REX101)
LAEAKVLANRELDKYGVSD**RYKNWINRAWLVALVKRQIDQILALLP** (SEQ. ID NO. 31) (REX107)
LAEAKVLANRELDKYGVSD**EYKNAINAANPVSGVKRPIDVILAALP** (SEQ. ID NO. 32) (REX108)
LAEAKVLANRELDKYGVSD**HYKNSINPAFKVHSVKMGIDWILAGLP** (SEQ. ID NO. 33) (REX127)
LAETKVLANRELDKYGVSD**WYKNRINTALTVACVKLVIDWILAALP** (SEQ. ID NO. 34) (REX136).

To characterize these polypeptide variants in more detail, we used competition ELISA with the immobilized H-exIL-23R and a constant amount of DH-MBP-p19 as an analyte, spiked with an increasing level of the REX-TolA-AVI variants. Maxisorp or Polysorp plates (NUNC, Denmark) were coated with recombinant H-exIL-23R or pelB-exIL-23R and binding of DH-MBP-p19 or DH-p19 as analytes was detected using antibodies as above. Alternatively, plates were coated with DH-MBP-p19 or DH-p19 and binding of IL-23R variants was detected using the corresponding antibodies. 20 nM DH-MBP-p19, 50 nM DH-p19 and 60 nM exIL-23R or pelB-exIL-23R in PBSTB were used as constant concentrations of analytes in the experiments where the concentration of the competitor REX binders varied by serial dilution. Three of the best inhibitory variants, REX125, REX115 and REX009, were examined for binding to immobilized recombinant human IL-23R-IgG chimera (R&D Systems, Minneapolis, MN), produced as soluble and glycosylated protein secreted by a mouse myeloma cell line. A Polysorp plate was coated with 1-2 μg/ml IL-23R-IgG chimera diluted in coating buffer, and 20 nM DH-MBP-p19 protein or 23 nM human IL-23 (R&D Systems, Minneapolis, MN) were used to compete with REX ligands.

We found that 11 of the 15 tested REX variants inhibited p19 binding in the micromolar to nanomolar concentration range. The results of the competition ELISA for the four best REX variants are presented in Figure 5. Sequence analysis of all tested variants indicated that two of the inhibitory variants, REX001 and REX009, contain one cysteine residue in the 11 randomized positions.

To further confirm the inhibitory potency of the REX binders, we performed competition ELISA with a different receptor-cytokine protein pair. REX inhibitors found in the previous setup of the competition ELISA also inhibited binding of the DH-p19 protein to the coated pelB-H-exIL-23R. In correlation with the result shown in Figure 5, all tested REX binders were found to suppress the p19 binding. To verify that the found competition potency of the tested REX clones can be attributed to the specific binding of the REX proteins to the refolded receptor molecule rather than to non-specific or misfolded protein binding, we performed additional competition assays with the same protein pairs but in the opposite ELISA layouts in which DH-MBP-p19 or DH-p19 were immobilized on the plastic plate. These results did confirm previous data.

To investigate whether inhibitory REX binders recognize the soluble and glycosylated form of IL-23R, we performed ELISA experiments with a commercial product of recombinant human IL-23R-IgG chimera (R&D Systems), secreted by an NSO-derived murine myeloma cell line. As shown in Figure 6a, increasing concentration of REX125, REX115 and REX009 binders decreased the binding of DH-MBP-p19 to the immobilized IL-23R-IgG. A similar inhibitory effect of the REX binders was also found for the recombinant Sf 21 (baculovirus)-derived human single-chain IL-23 (R&D Systems) (Fig. 6b). Thus, we conclude that we identified REX variants with the ability to suppress pl9/IL-23 binding to IL-23R, as demonstrated in several layouts of direct ELISA.

Example 10

**Fluorescence-based thermal-shift assay**

To investigate the thermal stability of the strongest inhibitory binders, we performed the fluorescence-based thermal shift assay (Fig.7). Protein samples (0.1 mg/ml) in HEPES, and 5x Sypro Orange dye (Sigma-Aldrich, St. Luis, MO) were added into 25 μl total volume. Using the real-time PCR Detection System CFX96 Touch (Bio-Rad Laboratories), the proteins were incubated in a thermal gradient from 20°C to 80°C at increments of 0.5°C and with 30 s-hold intervals. The degree of protein unfolding was monitored by the FRET (fluorescence resonance energy transfer) channel that captured the spectral properties of Sypro Orange unfolded protein complexes (excitation wavelength~470 nm and emission wavelength~570 nm). The data were analyzed by CFX Manager software and the melting temperatures were determined using the first derivative spectra.

Melting temperatures (Tm) for REX125 and REX128 were found to be 56 and 56.5°C, respectively, and for REX009 around 50°C, while the Tm value for WT control was found to be 58°C. This suggested that the randomization of mutable residues of the ABD domain did not significantly affect the basic stability of the scaffold structure.

Example 11

**Surface plasmon resonance measurement**

SPR biosensor binding analysis was used to further characterize the binding interactions between IL-23R and the REX ligands. Surface plasmon resonance measurements were carried out using custom SPR biosensors (Institute of Photonics and Electronics, Prague, Czech Republic) with four independent sensing spots (Vaisocherova H. et al., Biopolymers 2006;82(4):394-398). The $His_6$-REX-TolA-AVI and $His_6$-ABDwt-TolA-AVI proteins were immobilized to the SPR chip using the protocol described before (Sipova H. et al. Sensors Actuators B: Chem 2012;174(0):306-311). Briefly, the SPR sensor chip was coated with self-assembled monolayer of $HSC_{11}(EG)_2$-OH and $HSC_{11}(EG)_3OCH_2COOH$ alkenthiols. The functionalized SPR chip was mounted to the SPR sensor and all the subsequent molecular interactions were monitored in real-time at temperature 25°C and flow rate 30 μl/min. The REX009, REX125 and ABDwt proteins were diluted in SA buffer (10 mM sodium acetate, pH 5.0 at 25°C) at 5 μg/ml concentration. Each protein was immobilized to a separate sensing spot via amide-bond-forming chemistry. The non-covalently bound proteins were then washed away with a buffer of high ionic strength ($PBS_{Na}$; 1.4 mM $KH_2PO_4$, 8 mM $Na_2HPO_4$, 2.7 mM KCl, 0.75 M NaCl, pH 7.4 at 25°C). The remaining carboxylic groups were deactivated with 5-minute injection of 1M ethanolamine-hydrochloride (pH 8.5). After a baseline was established in the SA buffer, the solution of IL23-R was pumped in the sensor for 10 min. The measurement was repeated on three different chips. The interaction kinetics was compensated for reference sensor response (measured in channel with $ABD_{WT}$) and analyzed with BiaEvaluation software (GE Healthcare, Uppsala, Sweden).

The response to IL-23R-IgG chimera interaction of such SPR biosensor is shown in Figure 8 for sensor surfaces functionalized with REX125, REX009 and control ABDwt proteins, respectively. It can be seen that the sensor response to IL-23R-IgG was much higher in the channels coated with the REX proteins than in the reference channel functionalized with ABDwt. Interestingly, the interaction of IL-23R-IgG with the REX variants was very sensitive to pH and salt composition of the running buffer. The complex was stable at pH 5.0 of the sodium acetate (SA) buffer, while it was very rapidly dissociated at pH 7.4 in PBS buffer. Similar results were obtained when IL-23R-IgG was immobilized to the sensor surface and REX proteins were flowed over the sensor. The reference-compensated sensor responses to binding of IL-23R-IgG chimera to immobilized REX009, or REX125, respectively, at pH 5 were analyzed using an 1:1 interaction model in the kinetic BiaEvaluation software. Global fit of four concentrations in the range of 20-200nM indicated a dissociation constant $K_d$ = 1.3 ± 0.7 x $10^{-9}$ M for REX009. The binding affinity for REX125 could not be precisely determined due to a more complex interaction mode, but it was estimated to be in the order of $10^{-9}$ M.

Example 12

**Detection of cell surface IL-23R and binding of REX variants, p19 or IL-23 to the surface of cultured human cells**

To explore whether the REX ligands bind to the native IL-23 receptor molecules on the cell surface, we first investigated cultured human K-562 and Jurkat leukemic cells for the expression of IL-23R. All binding assays were performed in HBSS buffer (10 mM HEPES, pH 7.4, 140 mM NaCl, 5 mM KCl) complemented with 2 mM $CaCl_2$, 2 mM $MgCl_2$ and 1% (v/v) FCS (cHBSS) in 96-well culture plates (Nunc, Roskilde, Denmark). For staining of the IL-23R molecules on the cell surface, 5 x $10^5$ cells were incubated for 30 min at 4°C in 50 μl of cHBSS buffer containing anti-human IL-23R-APC (1:5 dilution) or isotype control-APC (1:5 dilution) mAbs. For REX binding assay, 5 x $10^5$ cells were incubated in 100 μl of cHBSS with biotinylated REX binders or ABDwt controls (10 μg/ml) for 30 min at 4°C, washed with cHBSS, and the cell-bound biotinylated REX-TolA-AVI or ABDwt-TolA-AVI was stained with streptavidin-phycoerythrin (dilution 1:400) for 30 min at 4°C. For DH-p19 binding assay, 5 x $10^5$ cells were incubated in 100 μl of cHBSS with or without DH-p19 (10 μg/ml) for 30 min at 4°C. The cells were washed with cHBSS and the cell-bound DH-p19 was stained with mouse anti-p19 mAb (1:50 dilution) for 30 min at 4°C and after washing with goat anti-mouse IgG antibody labeled with Cy5 (1:50 dilution) for 30 min at 4°C.

Cells were washed, resuspended in 100 μl of HBSS and analyzed by flow cytometry in a FACS LSR II instrument (BD Biosciences, San Jose, CA) in the presence of 5 μg/ml of propidium iodide. Appropriate gatings were used to exclude cell aggregates and dead cells and binding data were deduced from the mean fluorescence intensities (MFI).

We found that both K-562 and Jurkat cells express IL-23R and this expression is further increased upon T-cell activation, as documented 24 hours after the induction. We also found that THP-1 cells express more IL-23R than K-562, as documented in Figure 9a by IL-23R-specific polyclonal antibody and, therefore, both these cell lines were further used as targets for REX ligand-binding assay. As shown in Figure 9b, except for REX005, all of the tested REX variants bound to K-562 as well as THP-1 cells, and the extent of REX ligand binding correlated well with the observed level of IL-23R expression on the surface of both cell types (Fig. 9a). To further demonstrate that binding of REX ligands correlates with IL-23R expression, we performed a cell-surface binding test with Jurkat cells that express lower amounts of IL-23 receptor per cell than the K-562 cells (Fig. 9c). As shown in Figure 9d, all tested REX binders exhibited reduced binding to Jurkat cells in comparison to the K562 cells, in agreement with the reduced IL-23R expression on Jurkat cells. These results strongly suggested that the generated REX ligands can bind to native IL-23R molecules exposed on the human cell surface.

Example 13

**REX ligands competitively inhibit p19 subunit binding to the native IL-23 receptor on human cells**

To corroborate the observation that REX ligands bind to cell surface receptors, we performed assays in which the p19 protein was allowed to compete with REX ligands for the binding to IL-23R on the cell surface. For this purpose, we used THP-1 cells expressing higher levels of IL-23R (see Fig. 9a), to which also higher amounts of DH-p19 protein were bound than to K-562 cells (Fig. 10a). For blocking of REX binding to the IL-23 receptor molecule by DH-p19, THP-1 cells (2 x $10^5$) were preincubated for 15 min at 4 °C in the presence of serially-diluted DH-p19 in 50 μl of cHBSS buffer. Biotinylated REX-TolA-AVI clones (or ABDwt-TolA-AVI negative control) were added to the cells in the continuous presence of DH-p19 in 50 μl of cHBSS buffer to a final concentration of 13 nM and incubated at 4°C for 30 min. The cells were washed with cHBSS and the bound biotinylated REX ligands or ABDwt were detected by streptavidin-phycoerythrin conjugate (dilution 1:400) for 30 min at 4 °C. Cells were washed, resuspended in 100 μl of HBSS and analyzed by flow cytometry as described above. As shown in Figure 10b, at increasing concentrations of the DH-p19 protein, the binding of all three tested REX ligands to THP-1 cells was significantly inhibited.

Example 14

**REX binders inhibit IL-23-dependent *ex vivo* expansion of IL-17-positive CD4+ T-cells**

The sum of the above-outlined results indicated that at least several of the generated REX ligands could bind to native IL-23R molecules on human cells and block the p19-subunit-mediated binding of the IL-23 cytokine to its receptor. Therefore, we assessed *ex vivo* whether the REX ligands could inhibit the IL-23 signaling function on primary human T-cells. We determined whether blocking of IL-23R by excess of REX ligands would inhibit IL-23-mediated expansion of primary human Th-17 lymphocytes. Peripheral blood of healthy donors drawn into EDTA-containing tubes was used to obtain purified mononuclear cells (PBMCs) on Ficoll-Paque gradients (Pharmacia, Uppsala, Sweden). PBMCs were washed once with PBS and resuspended in complete RPMI 1640 media (RPMI 1640 supplemented with 10% heat-inactivated FCS, 100 U/ml penicillin, 100 μg/ml streptomycin sulfate and 1.7 mM sodium glutamate). PBMCs were adjusted to 2x $10^6$ cells/ml and activated on a 96-well plate pre-coated with anti-CD3 (MEM-57, 10 μg/ml, Exbio Praha a.s., Praha, Czech Republic) in the presence of co-stimulatory antibodies against CD28 and CD49d (1 μg/ml, BD Biosciences, San Jose, CA, USA), in the presence of IL-23 (10 ng/ml) and IL-2 (100 U/ml). REX009, REX115, REX125, REX128 binders (7 μg/ml) or control ABDwt were added and cells were incubated for three days at 37°C. After overnight rest at 37°C, cells were re-stimulated again (as above) for 6 h at 37°C (last 4 hours exocytosis was blocked with Brefeldin A (10 μg/ml, Sigma-Aldrich). Next, cells were stained with antibodies to CD8 Horizon V-500 (BDB) for 15 min in the dark, washed with PBS containing 0.1% sodium azide and 2% gelatin from cold fish (Sigma-Aldrich) and fixed using FACS lysing solution/FACS Perm 2 (BDB) according to the manufacturer's instructions. Upon further washing, cells were next stained for CD3 (PerCP-Cy5.5, eBioscience, San Diego, CA, USA), for CD4 (ECD, Immunotech, Marseille, France) and with antibodies for IFN-γ (PE Cy7), IL-2 (APC), IL-17 (PB) (eBioscience) and CD 154 (PE, Immunotech). Cells were washed again and measured in flow cytometr BD FACS Aria III (BDB). Absolute cell counts were obtained using BD Truecount (BDB). Viable, nucleated cells were counterstained with Syto-16 and DAPI (Invitrogen).

As shown in Figure 11, over the three days of *ex vivo* cultivation of PBMC suspensions, a marked decrease of Th-17 cell expansion (decrease of counts of IL-17-secreting, IFN-γ non-secreting T-cells) was observed when excess of the REX ligands was present, as compared to mock treatment or presence of the ABD-WT control. In PBMC suspensions, the enhancement of Th17+ cells after IL-23-mediated induction is documented as a difference between non-induced cells (sample NO IL-23, 0.52±0.11, n=6, p=0.0001) and normalized counts of IL-23 stimulation (IL-23 only, =1), shown as a boxplot in Figure 11b. The addition of ABD-WT as a control into the PBMC suspensions had little effect on Th-17+ cell expansion (0.88±0.26, n=7, p=0.2892). In contrast, all tested REX variants significantly inhibited Th-17+ cell expansion: REX009 (0.62±0.32, n=7, p=0.0559), REX115 (0.55±0.20, n=5, p=0.0074) and REX125 (0.42±0.17, n=5, p=0.0004), as documented in Figure 11b.

To verify whether the observed inhibitory effect of REX binders in PBMC suspensions can be attributed to the T-cell-dependent cell expansion, we repeated the same experiment in samples with separated T-cells isolated from the same PBMC donors. As shown in Figure 11c, the IL-23-dependent Th-17+ cell count enrichment was also detected (NO IL-23, 0.77±0.08, n=4, p=0.0116). T-cell suspensions with added ABD-WT control exhibited no inhibitory effect on T-cell expansion (0.99±0.26, n=4, p=0.9901). In striking contrast to this observation, REX variants demonstrated an immunosuppressive effect on T-cell expansion as follows: REX115 (0.69±0.17, n=3, p=0.0883), REX125 (0.65±0.16, n=3, p=0.0671) and REX009 (0.80±0.01, n=2, p=0.0139).

In summary, the collected data indicate that the tested REX variants inhibited IL-23-dependent Th-17+ cell expansion to the level of no-IL-23-induced samples (REX009) or even below this level (REX115 and REX125) and this finding is valid for both PBMC and separated T-cell samples. While IL-23 was shown to promote development of Th-17+ T-cells in man (Acosta-Rodriguez EV et al. Nat Immunol 2007;8: 942-9), the mode of its action might be fixing the Th-17 commitment rather than "de novo" Th-17 development, survival or proliferation enhancement (Stritesky GL, Yeh N, Kaplan MH. J Immunol 2008;181:5948-5955). As shown here, REX ligands might block the IL-23 signaling function on human T-cells through selective binding and occupation of the IL-23 receptor.

## Claims

**1.** A polypeptide, **characterized in that** it contains a sequence with at least 80% identity with at least one sequence selected from the group comprising:

KYKNGINNALCVRRVKALIDWILAYLP (SEQ. ID NO. 1)
TYKNDINAASYVPAVKWAIDRILASLP (SEQ. ID NO. 2)
YYKNRINPACHVLSVKSNIDWILASLP (SEQ. ID NO. 3)
VYKNTINIAIPVRVVKRVIDWILAVLP (SEQ. ID NO. 4)
AYKNLINAALIVAKVKLLIDAILAPLP (SEQ. ID NO. 5)
HYKNWINPARRVRPVKWLIDAILAALP (SEQ. ID NO. 6)
RYKNSINRALPVAAVKWALDLILAWLP (SEQ. ID NO. 7)
WYKNCITAARAVTTVKLLIDTILALLP (SEQ. ID NO. 8)
HYKNPINVAWTVGRVKVWIDAILAPLP (SEQ ID NO. 9)
PYKNPINCACPVTEVKPPIDAILALLP (SEQ. ID NO. 10)
HYKNSINPAPQVIVVKVNIDLILAGLP (SEQ. ID NO. 11)
RYKNWINRAWLVALVKRQIDQILALLP (SEQ. ID NO. 12)
EYKNAINAANPVSGVKRPIDVILAALP (SEQ. ID NO. 13)
HYKNSINPAFKVHSVKMGIDWILAGLP (SEQ. ID NO. 14)
WYKNRINTALTVACVKLVIDWILAALP (SEQ. ID NO. 15)

whereas at the C-terminus or at the N-terminus of said sequence is attached a sequence having at least 80% identity with at least one sequence selected from the group comprising:

LAEAKVLANRELDKYGVSD (SEQ ID NO. 17)
LAEAKVLTNRELDKYGVSD (SEQ ID NO. 18)
LAETKVLANRELDKYGVSD (SEQ ID NO. 19),

and wherein the affinity binding constant for binding of said polypeptide to IL-23 receptor has a value of less than $10^{-7}$ M.

2. A DNA sequence, **characterized in that** it is selected from the group comprising complementary DNA coding for the amino acid sequence of the polypeptides of claim 1, and DNA hybridizing with said complementary DNA under conditions of high stringency.

3. Use of the DNA sequence of claim 2 for the preparation of polypeptides or recombinant proteins produced in bacterial, yeast, insect, mammal or human host cells.

4. A host cell, **characterized in that** it contains at least one DNA sequence according to claim 2.

5. A pharmaceutical composition comprising polypeptide according to claim 1 and a pharmaceutically acceptable carrier.

6. The polypeptide according to claim 1 for use in medicine.

7. The polypeptide according to claim 1 for use in the treatment of autoimmune diseases.

8. The polypeptide according to claim 1 for use in the treatment of an autoimmune disease selected from the group comprising psoriasis, psoriatic arthritis, Crohn’s disease, rheumatoid arthritis and multiple sclerosis.

9. The polypeptide according to claim 1 for use as a diagnostic.

## Patentansprüche

1. Polypeptid **dadurch gekennzeichnet, dass** es eine Sequenz enthält, die eine mindestens 80%-Sequenzidentität mit mindestens einer aus der folgenden Gruppe ausgewählten Sequenz aufweist:

KYKNGINNALCVRRVKALIDWILAYLP (SEQ. ID-Nr. 1)
TYKNDINAASYVPAVKWAIDRILASLP (SEQ. ID-Nr. 2)
YYKNRINPACHVLSVKSNIDWILASLP (SEQ. ID-Nr. 3)
VYKNTINIAIPVRVVKRVIDWILAVLP (SEQ. ID-Nr. 4)
AYKNLINAALIVAKVKLLIDAILAPLP (SEQ. ID-Nr. 5)
HYKNWINPARRVRPVKWLIDAILAALP (SEQ. ID-Nr. 6)
RYKNSINRALPVAAVKWALDLILAWLP (SEQ. ID-Nr. 7)
WYKNCITAARAVTTVKLLIDTILALLP (SEQ. ID-Nr. 8)
HYKNPINVAWTVGRVKVWIDAILAPLP (SEQ ID-Nr. 9)
PYKNPINCACPVTEVKPPIDAILALLP (SEQ. ID-Nr. 10)
HYKNSINPAPQVIVVKVNIDLILAGLP (SEQ. ID-Nr. 11)
RYKNWINRAWLVALVKRQIDQILALLP (SEQ. ID-Nr. 12)
EYKNAINAANPVSGVKRPIDVILAALP (SEQ. ID-Nr. 13)
HYKNSINPAFKVHSVKMGIDWILAGLP (SEQ. ID-Nr. 14)
WYKNRINTALTVACVKLVIDWILAALP (SEQ. ID-Nr. 15),

wobei am C-Ende oder am N-Ende der angeführten Sequenz eine Sequenz angeschlossen ist, die eine mindestens 80%-Sequenzidentität mit mindestens einer aus der folgenden Gruppe ausgewählten Sequenz aufweist:

LAEAKVLANRELDKYGVSD (SEQ ID NO. 17)
LAEAKVLTNRELDKYGVSD (SEQ ID NO. 18)
LAETKVLANRELDKYGVSD (SEQ ID NO. 19),

und wobei die affinite Bindungskonstante für die Bindung des genannten Polypeptids zum Rezeptor IL-23 einen Wert geringer als $10^{-7}$ M aufweist.

2. DNA-Sequenz **dadurch gekennzeichnet, dass** sie aus der Gruppe ausgewählt ist, umfassend eine komplementäre DNA kodierend für die Aminosäuresequenz von Polypeptiden nach dem Anspruch 1 und eine DNA hybridisierend mit der genannten komplementären DNA unter stringenten Bedingungen.

3. Verwendung der DNA-Sequenz nach dem Anspruch 2 zur Herstellung von Polypeptiden oder von rekombinanten Proteinen produziert in Bakterien-, Hefen-, Insekten-, Säuger- oder menschlichen Gastgeberzellen.

4. Gastgeberzelle **dadurch gekennzeichnet, dass** sie mindestens eine DNA-Sequenz nach dem Anspruch 2 einschliesst.

5. Pharmazeutische Komposition enthaltend das Polypeptid nach dem Anspruch 1 und einen pharmazeutisch annehmbaren Träger.

6. Polypeptid nach dem Anspruch 1 zur Verwendung in der Medizin.

7. Polypeptid nach dem Anspruch 1 zur Verwendung bei der Behandlung von Autoimmunkrankheiten.

8. Polypeptid nach dem Anspruch 1 zur Verwendung bei der Behandlung einer Autoimmunkrankheit ausgewählt aus der Gruppe umfassend Psoriasis, Psoriasisarthritis, Morbus Crohn, rheumatoide Arthritis und Multiple Sklerose.

9. Polypeptid nach dem Anspruch 1 zur Verwendung als Diagnosemittel.

## Revendications

1. Polypeptide, **caractérisé en ce qu'**il contient une séquence présentant au moins 80 % d'identité avec la séquence choisie parmi le groupe constitué par :

   KYKNGINNALCVRRVKALIDWILAYLP (SEQ. ID NO. 1)
   TYKNDINAASYVPAVKWAIDRILASLP (SEQ. ID NO. 2)
   YYKNRINPACHVLSVKSNIDWILASLP (SEQ. ID NO. 3)
   VYKNTINIAIPVRVVKRVIDWILAVLP (SEQ. ID NO. 4)
   AYKNLINAALIVAKVKLLIDAILAPLP (SEQ. ID NO. 5)
   HYKNWINPARRVRPVKWLIDAILAALP (SEQ. ID NO. 6)
   RYKNSINRALPVAAVKWALDLILAWLP (SEQ. ID NO. 7)
   WYKNCITAARAVTTVKLLIDTILALLP (SEQ. ID NO. 8)
   HYKNPINVAWTVGRVKVWIDAILAPLP (SEQ ID NO. 9)
   PYKNPINCACPVTEVKPPIDAILALLP (SEQ. ID NO. 10)
   HYKNSINPAPQVIVVKVNIDLILAGLP (SEQ. ID NO. 11)
   RYKNWINRAWLVALVKRQIDQILALLP (SEQ. ID NO. 12)
   EYKNAINAANPVSGVKRPIDVILAALP (SEQ. ID NO. 13)
   HYKNSINPAFKVHSVKMGIDWILAGLP (SEQ. ID NO. 14)
   WYKNRINTALTVACVKLVIDWILAALP (SEQ. ID NO. 15)

   dans lequel sur l'extrémité C-terminale ou sur l'extrémité N-terminale de la séquence indiquée est attachée une séquence présentant au moins 80 % d'identité avec au moins une séquence choisie parmi le groupe constitué par :

   LAEAKVLANRELDKYGVSD (SEQ ID NO. 17)
   LAEAKVLTNRELDKYGVSD (SEQ ID NO. 18)
   LAETKVLANRELDKYGVSD (SEQ ID NO. 19),

   où la constante d'affinité pour la liaison du polypeptide indiqué à son récepteur IL-23 présente une valeur inférieure à $10^{-7}$ M.

2. La séquence ADN, **caractérisée en ce qu'**elle est choisie parmi le groupe constitué par l'ADN complémentaire codant la séquence d'acides aminés de polypeptides selon la revendication 1 et l'ADN hybridant avec l'ADN complémentaire indiqué sous des conditions de forte stringence.

**3.** L'utilisation de la séquence ADN selon la revendication 2 pour la préparation de polypeptides ou de protéines recombinants produits dans les cellules hôtes bactériennes, de levure, d'insecte, de mammifères ou humaines.

**4.** Cellule hôte, **caractérisée en ce qu'**elle contient au moins une séquence ADN selon la revendication 2.

**5.** Composition pharmaceutique contenant le polypeptide selon la revendication 1 et un véhicule pharmaceutiquement acceptable.

**6.** Polypeptide selon la revendication 1 pour utilisation en médecine.

**7.** Polypeptide selon la revendication 1 pour utilisation dans le traitement de maladies auto-immunes.

**8.** Polypeptide selon la revendication 1 pour utilisation dans le traitement de la maladie autoimmune choisie parmi le groupe constitué par le psoriasis, l'arthrite psoriasique, la maladie de Crohn, l'arthrite rhumatoïde et la sclérose en plaques.

**9.** Polypeptide selon la revendication pour utilisation comme agent diagnostique.

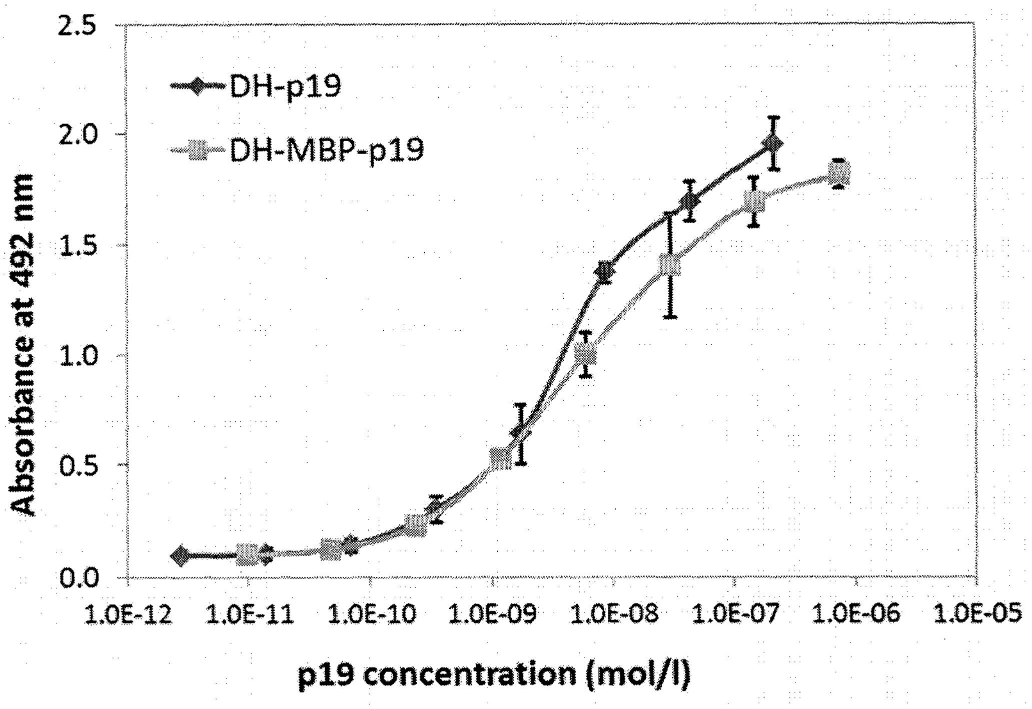
2.5
2.0
1.5
1.0
0.5
0.0
DH-p19
DH-MBP-p19
Absorbance at 492 nm
1.0E-12
1.0E-11
1.0E-10
1.0E-09
1.0E-08
1.0E-07
1.0E-06
1.0E-05
p19 concentration (mol/l)

Fig. 1

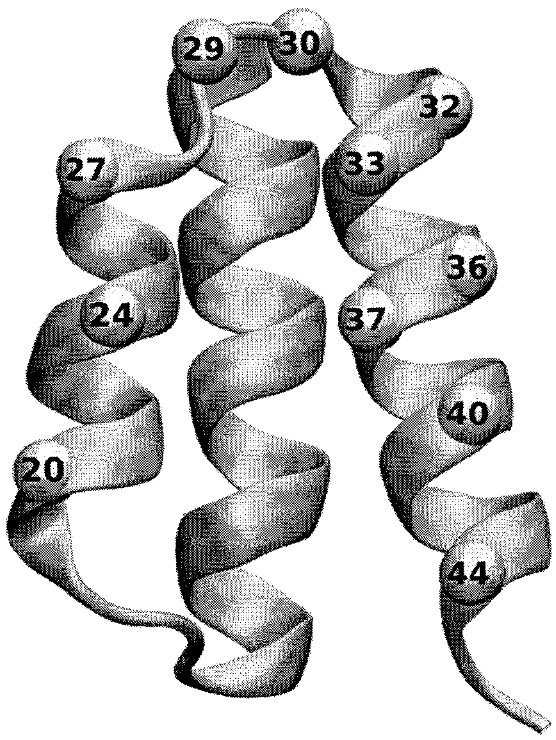
29
30
32
27
33
36
24
37
40
20
44

Fig. 2

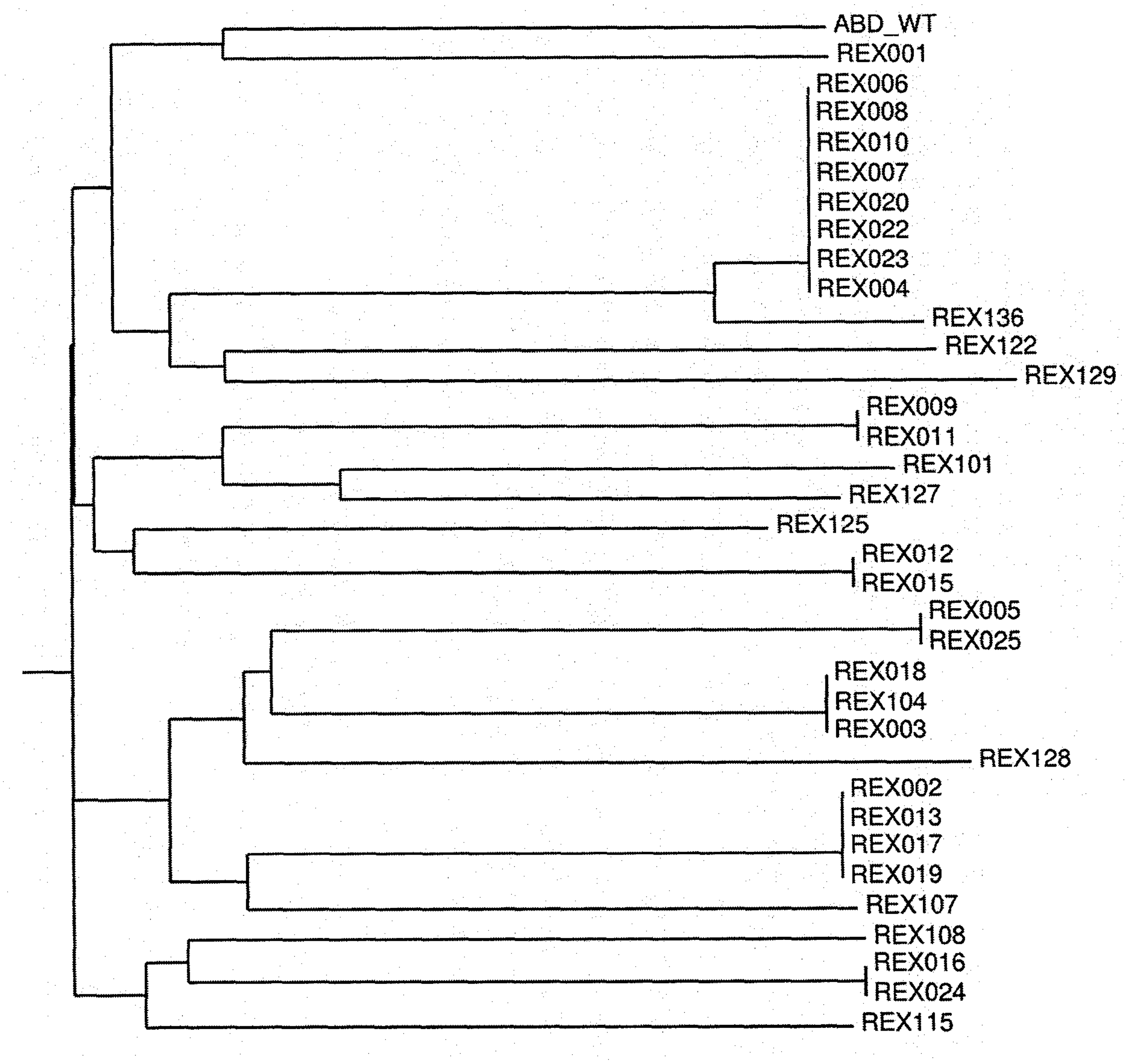
ABD_WT
REX001
REX006
REX008
REX010
REX007
REX020
REX022
REX023
REX004
REX136
REX122
REX129
REX009
REX011
REX101
REX127
REX125
REX012
REX015
REX005
REX025
REX018
REX104
REX003
REX128
REX002
REX013
REX017
REX019
REX107
REX108
REX016
REX024
REX115

Fig. 3

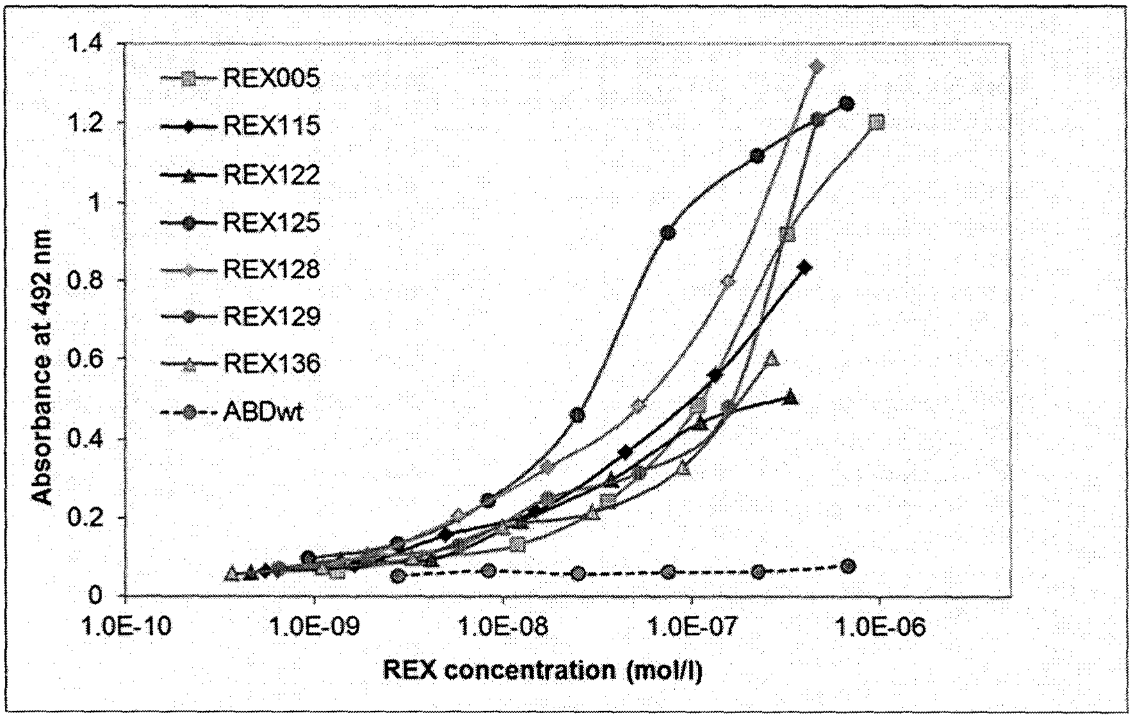
1.4
1.2
1
0.8
0.6
0.4
0.2
0
Absorbance at 492 nm
REX005
REX115
REX122
REX125
REX128
REX129
REX136
ABDwt
1.0E-10
1.0E-09
1.0E-08
1.0E-07
1.0E-06
REX concentration (mol/l)

Fig. 4a

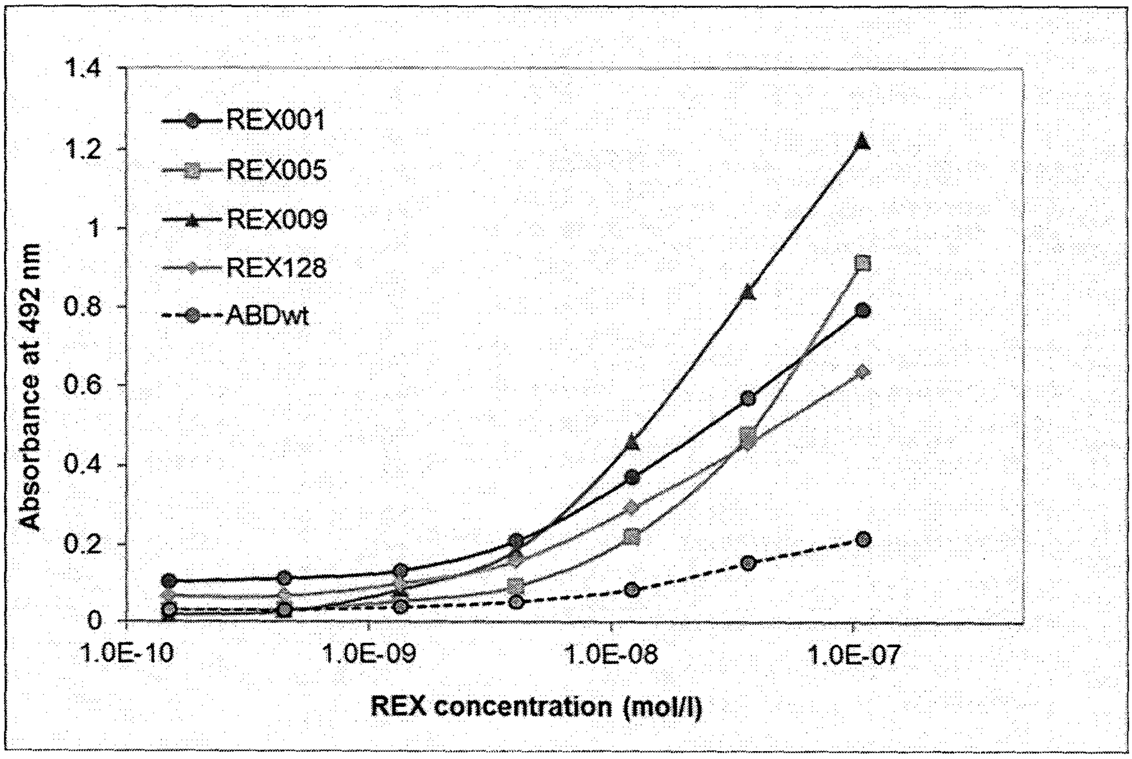
1.4
1.2
1
0.8
0.6
0.4
0.2
0
Absorbance at 492 nm
REX001
REX005
REX009
REX128
ABDwt
1.0E-10
1.0E-09
1.0E-08
1.0E-07
REX concentration (mol/l)

Fig. 4b

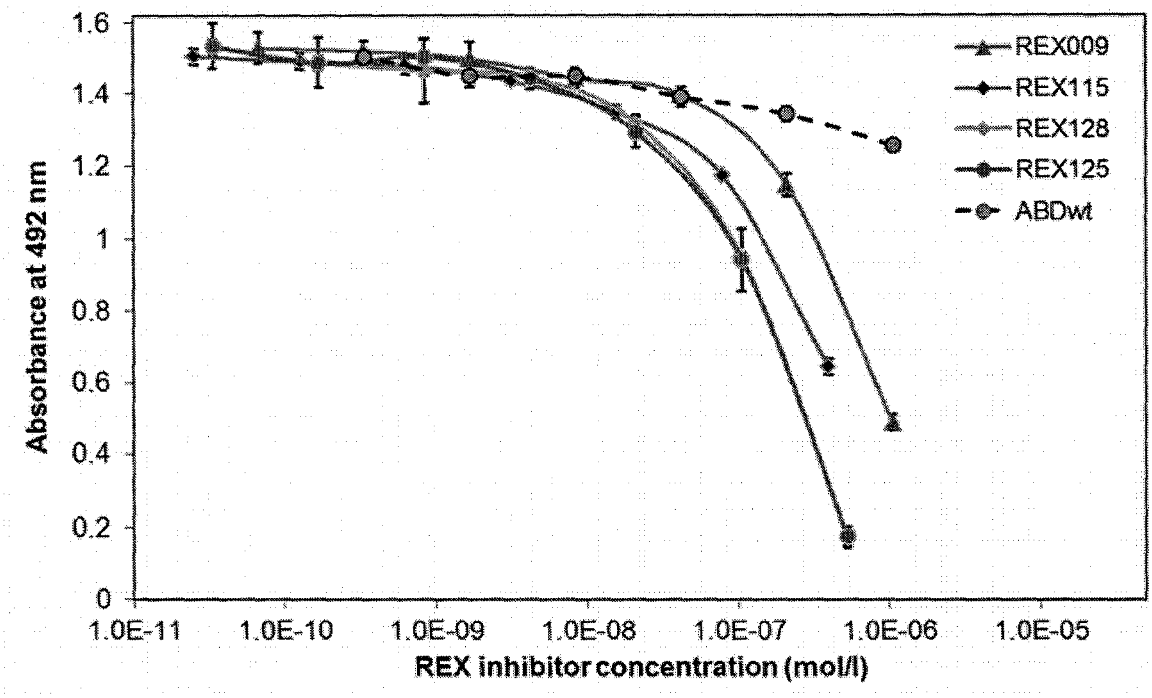
1.6
1.4
1.2
1
0.8
0.6
0.4
0.2
0
Absorbance at 492 nm
1.0E-11
1.0E-10
1.0E-09
1.0E-08
1.0E-07
1.0E-06
1.0E-05
REX inhibitor concentration (mol/l)
REX009
REX115
REX128
REX125
ABDwt

Fig. 5

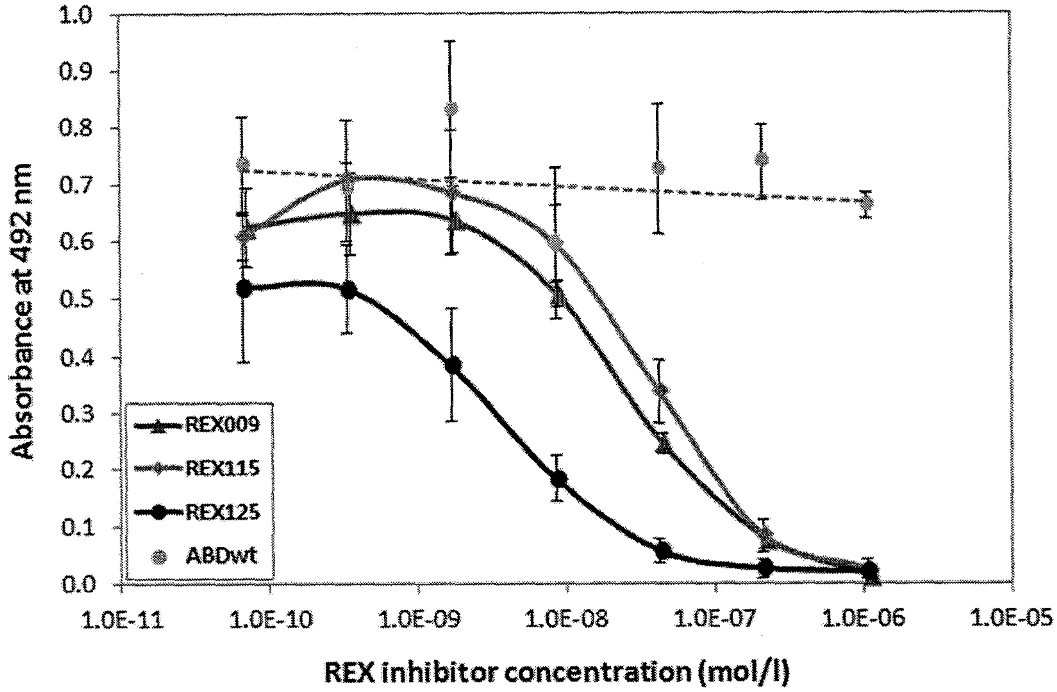

1.0
0.9
0.8
0.7
0.6
0.5
0.4
0.3
0.2
0.1
0.0
Absorbance at 492 nm
REX009
REX115
REX125
ABDwt
1.0E-11
1.0E-10
1.0E-09
1.0E-08
1.0E-07
1.0E-06
1.0E-05
REX inhibitor concentration (mol/l)


Fig. 6a

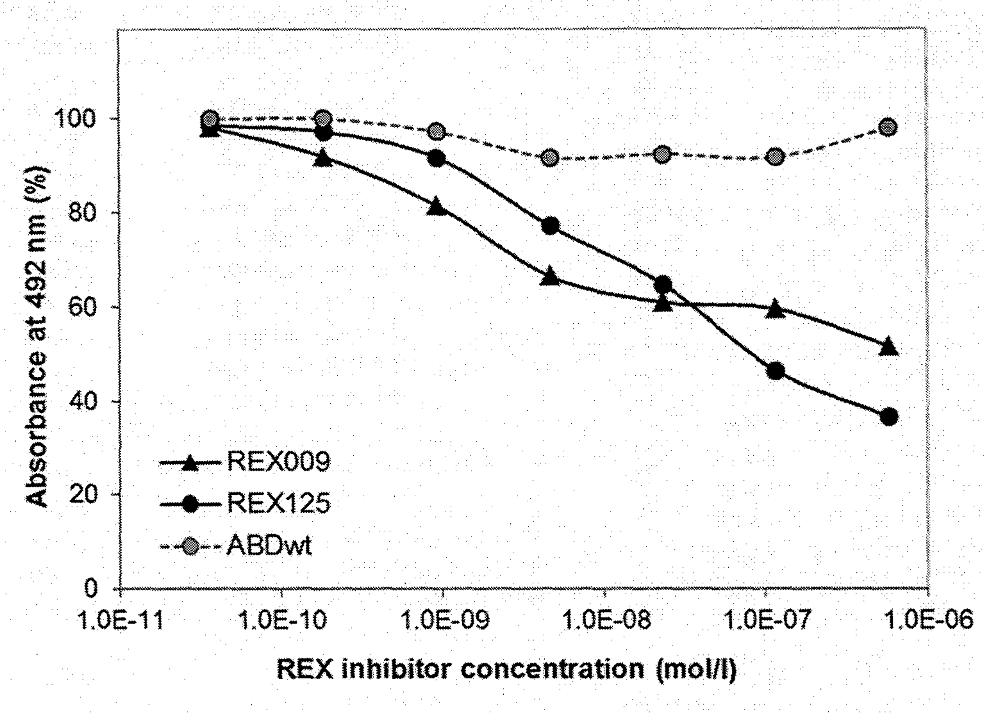

100
80
60
40
20
0
Absorbance at 492 nm (%)
REX009
REX125
ABDwt
1.0E-11
1.0E-10
1.0E-09
1.0E-08
1.0E-07
1.0E-06
REX inhibitor concentration (mol/l)


Fig. 6b

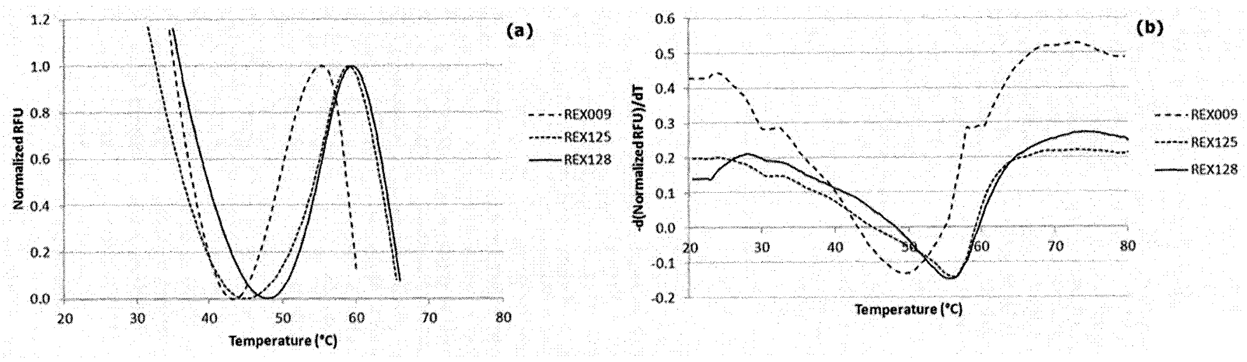
(a)
Normalized RFU
Temperature (°C)
REX009
REX125
REX128
(b)
-d(Normalized RFU)/dT
Temperature (°C)
REX009
REX125
REX128

Fig. 7

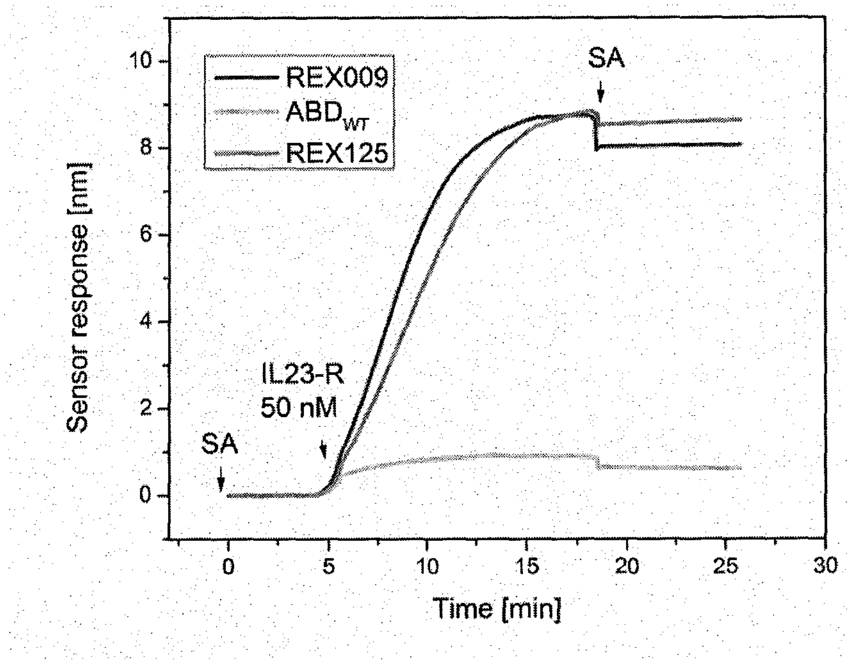
REX009
ABD_WT
REX125
SA
IL23-R
50 nM
SA
Sensor response [nm]
Time [min]
0
2
4
6
8
10
0
5
10
15
20
25
30

Fig. 8

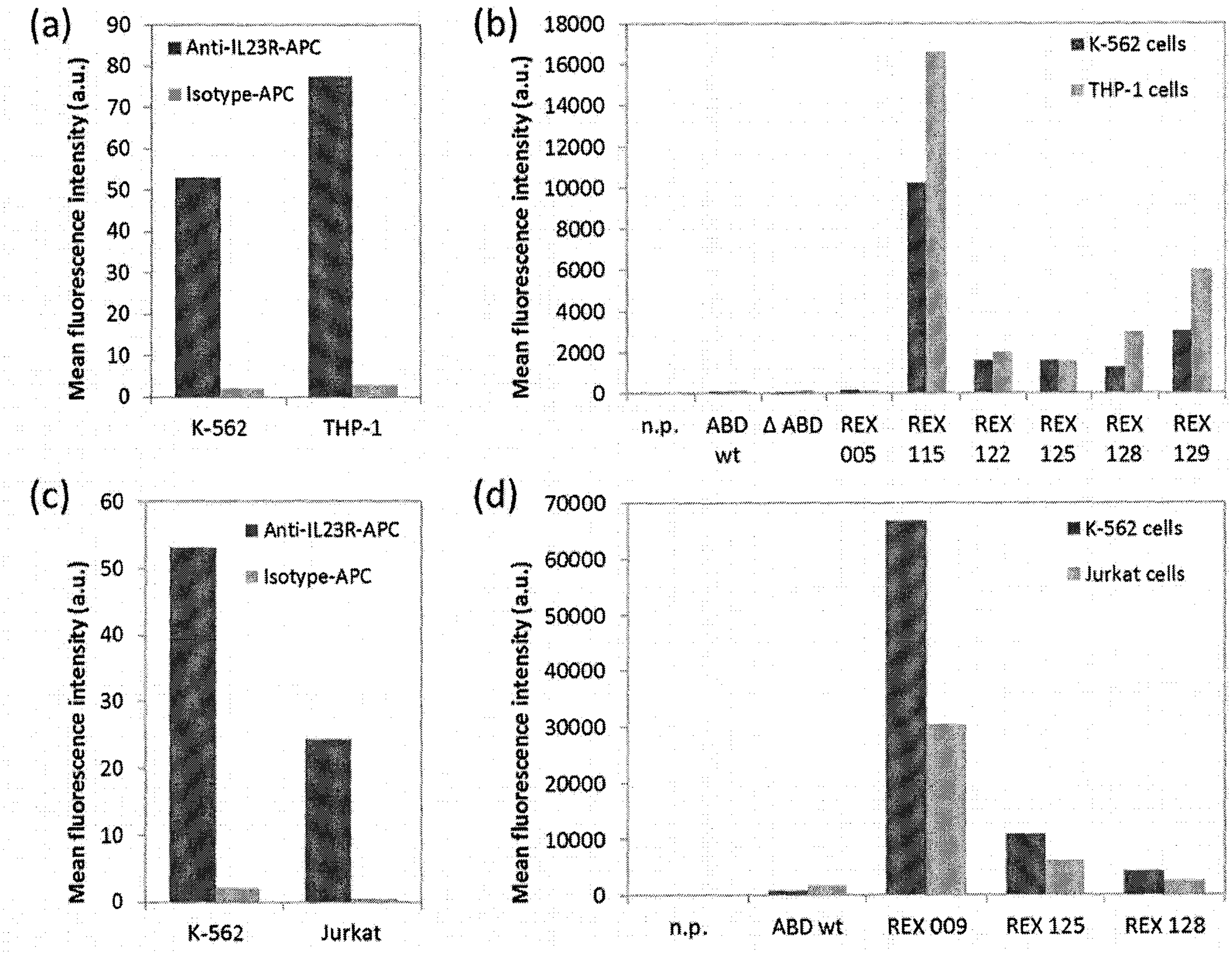
(a)
Mean fluorescence intensity (a.u.)
90
80
70
60
50
40
30
20
10
0
Anti-IL23R-APC
Isotype-APC
K-562
THP-1
(b)
Mean fluorescence intensity (a.u.)
18000
16000
14000
12000
10000
8000
6000
4000
2000
0
K-562 cells
THP-1 cells
n.p.
ABD wt
Δ ABD
REX 005
REX 115
REX 122
REX 125
REX 128
REX 129
(c)
Mean fluorescence intensity (a.u.)
60
50
40
30
20
10
0
Anti-IL23R-APC
Isotype-APC
K-562
Jurkat
(d)
Mean fluorescence intensity (a.u.)
70000
60000
50000
40000
30000
20000
10000
0
K-562 cells
Jurkat cells
n.p.
ABD wt
REX 009
REX 125
REX 128

Fig. 9

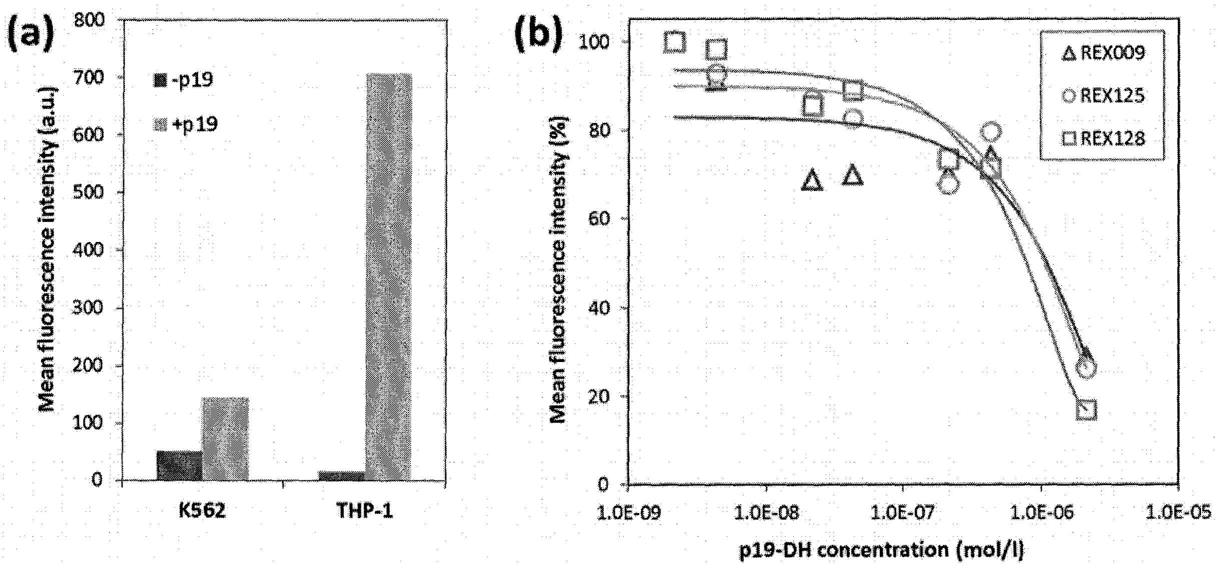
(a)
Mean fluorescence intensity (a.u.)
0
100
200
300
400
500
600
700
800
-p19
+p19
K562
THP-1
(b)
Mean fluorescence intensity (%)
0
20
40
60
80
100
REX009
REX125
REX128
1.0E-09
1.0E-08
1.0E-07
1.0E-06
1.0E-05
p19-DH concentration (mol/l)

Fig. 10

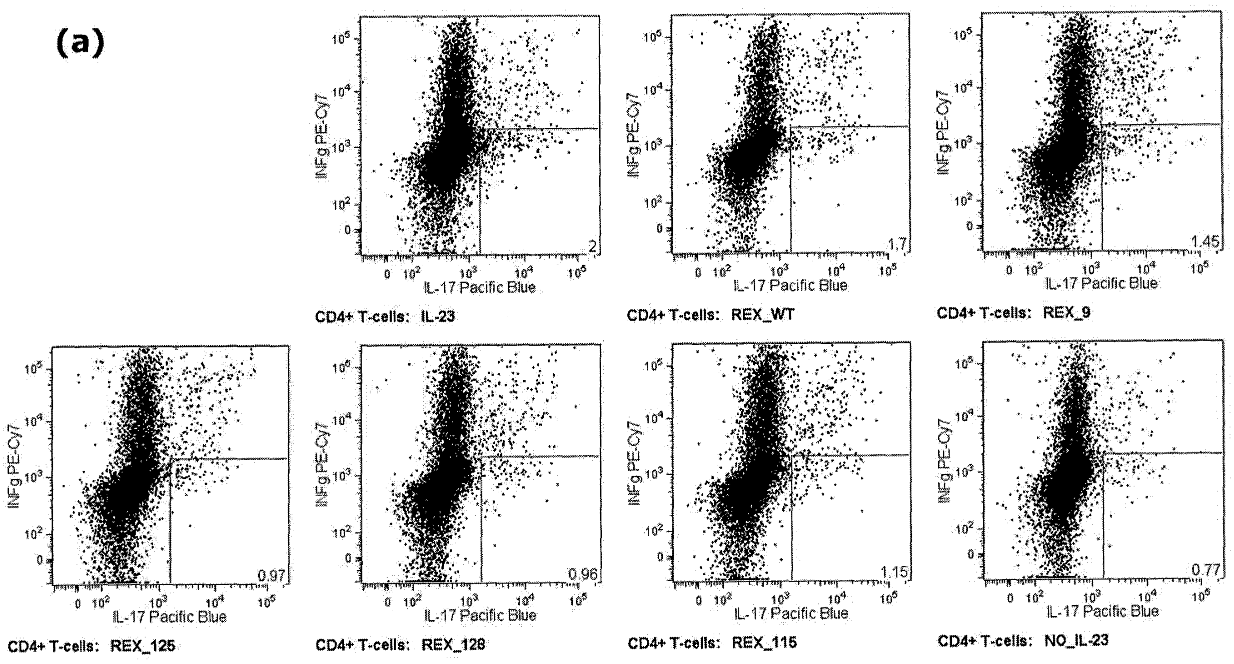
(a)
INFg PE-Cy7
IL-17 Pacific Blue
2
CD4+ T-cells: IL-23
1.7
CD4+ T-cells: REX_WT
1.45
CD4+ T-cells: REX_9
0.97
CD4+ T-cells: REX_125
0.96
CD4+ T-cells: REX_128
1.15
CD4+ T-cells: REX_115
0.77
CD4+ T-cells: NO_IL-23

Fig. 11

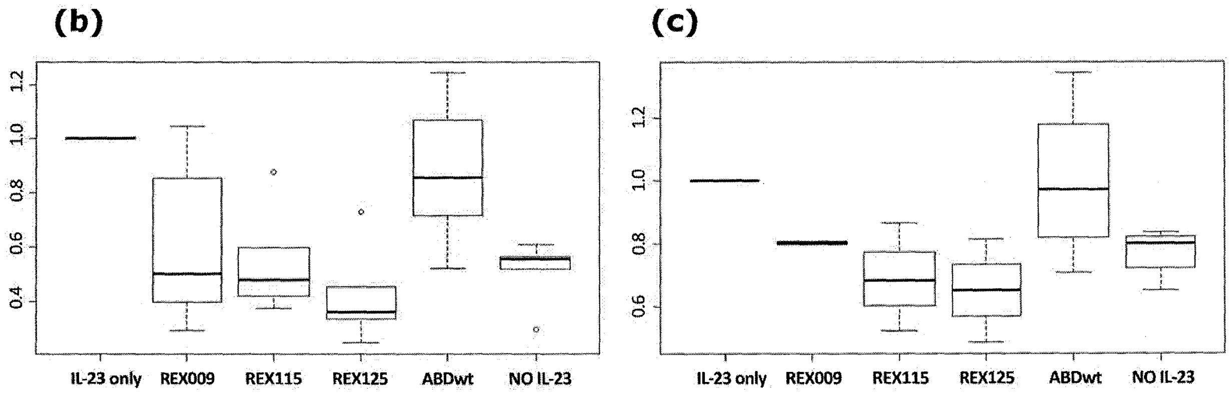
(b)
1.2
1.0
0.8
0.6
0.4
IL-23 only
REX009
REX115
REX125
ABDwt
NO IL-23
(c)
1.2
1.0
0.8
0.6
IL-23 only
REX009
REX115
REX125
ABDwt
NO IL-23

Fig. 11 – cont.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008103432 A
- WO 2010027766 A
- WO 2012093127 A
- US 20080187517 A
- WO 2011103105 A


### Non-patent literature cited in the description


- **OPPMANN B et al.** *Immunity,* 2000, vol. 13 (5), 715-725
- **CUA DJ et al.** *Nature,* 2003, vol. 421 (6924), 744-748
- **LANGRISH CL et al.** *J Exp Med,* 2005, vol. 201 (2), 233-240
- **CHAN JR et al.** *J Exp Med,* 2006, vol. 203 (12), 2577-2587
- **CAPON F et al.** *Hum Genet,* 2007, vol. 122 (2), 201-206
- **VAKNIN-DEMBINSKY A et al.** *J Immunol,* 2006, vol. 176 (12), 7768-7774
- **DUERR RH et al.** *Science,* 2006, vol. 314 (5804), 1461-1463
- **BEYER BM et al.** *J Mol Biol,* 2008, vol. 382 (4), 942-955
- **LUPARDUS PJ et al.** *J Mol Biol,* 2008, vol. 382 (4), 931-941
- **PARHAM C et al.** *J Immunol,* 2002, vol. 168 (11), 5699-5708
- **KASTELEIN RA et al.** *Annu Rev Immunol,* 2007, vol. 25, 221-242
- **BONIFACE K et al.** *Immunol Rev,* 2008, vol. 226, 132-146
- **TOICHI E et al.** *J Immunol,* 2006, vol. 177 (7), 4917-4926
- **KRUEGER GG et al.** *N Engl J Med,* 2007, vol. 356 (6), 580-592
- **GOTTLIEB AB et al.** *Curr Med Res Opin,* 2007, vol. 23 (5), 1081-1092
- **LEONARDI CL et al.** *Lancet,* 2008, vol. 371 (9625), 1665-1674
- **PAPP KA et al.** *Lancet,* 2008, vol. 371 (9625), 1675-1684
- **GARBER K.** *Nat Biotechnol,* 2012, vol. 30 (6), 475-477
- *Current Opinion in Biotechnology,* 2005, vol. 16 (4), 459-469
- **NYGREN PA et al.** *Journal of Immunological Methods,* 2004, vol. 290 (1-2), 3-28
- **GRONWALL C et al.** *J Biotechnol,* 2009, vol. 140 (3-4), 254-269
- **AHMAD JN et al.** *Proteins,* 2012, vol. 80 (3), 774-789
- **VAISOCHEROVA H. et al.** *Biopolymers,* 2006, vol. 82 (4), 394-398
- **SIPOVA H. et al.** *Sensors Actuators B: Chem,* 2012, vol. 174 (0), 306-311
- **ACOSTA-RODRIGUEZ EV et al.** *Nat Immunol,* 2007, vol. 8, 942-9
- **STRITESKY GL ; YEH N ; KAPLAN MH.** *J Immunol,* 2008, vol. 181, 5948-5955